# EUROPEAN PATENT SPECIFICATION

(11) **EP 2 358 472 B1**
(45) Date of publication and mention of the grant of the patent: **17.04.2013**
(21) Application number: 09826366.8
(22) Date of filing: 06.11.2009
(51) Int. Cl.: B01L 3/00, B81B 7/00, C12M 1/16, C12M 3/00

(54) **FLUIDIC CULTURE DEVICE**
FLÜSSIGKEITSKULTURVORRICHTUNG
DISPOSITIF DE CULTURE FLUIDIQUE

(30) Priority: 17.11.2008 US 115124 P
(43) Date of publication of application: 24.08.2011
(73) Proprietor: Gradientech Ab, 753 18 Uppsala (SE)
(72) Inventor: KREUGER, Johan, S-756 47 Uppsala (SE); THORSLUND, Sara, S-753 18 Uppsala (SE); BARKEFORS, Irmeli, S-753 18 Uppsala (SE)
(74) Representative: Sjölander, Henrik
(86) International application number: PCT/SE2009/051270
(87) International publication number: WO 2010/056186

(56) References cited:
- WO-A2-2007/124481
- FR-A1- 2 861 608
- US-A1- 2006 240 548
- FRISK T ET AL: "A microfluidic device for parallel 3-D cell cultures in asymmetric environments", ELECTROPHORESIS, WILEY INTERSCIENCE, DE, vol. 28, 1 January 2007 (2007-01-01), pages 4705-4712, XP003026409, ISSN: 0173-0835, DOI: 10.1002/ALPS.200700342
- FRISK T. ET AL: 'A microfluidic device for parallel 3-D cell cultures in asymmetric environments' ELECTROPHORESIS vol. 28, 2007, pages 4705 - 4712, XP003026409

## Description

### TECHNICAL FIELD

The present invention generally relates to culture devices, and in particular to fluidic culture devices for cell, tissue and/or organ investigation.

### BACKGROUND

The traditional approach of cell biological studies has mainly been limited to Petri dishes and microscope slides. Such two-dimensional (2D) culture methods, though, have several limitations. For instance, cells grown in 2D cultures often exhibit different behavior as compared to the natural organization of cells into three-dimensional (3D) patters surrounded by other cells as well as extracellular matrix (ECM). Additionally, cells communicate via secretion of signaling molecules. These signaling molecules have the ability to form instructive gradients in tissue, and it is therefore vital for cells to be able to interpret and respond to different types of molecular gradients. Gradients are hard to form and control in the traditional 2D culture methods.

Conventional assays generally used to study cell migration in response to molecular gradients include the Boyden chamber assay, as well as a micropipette-based assay for gradient formation, and the under-agarose assay. These assays do not support the formation of stable gradients over long periods of time, and may be difficult to combine with high resolution real-time imaging of cell responses. Nevertheless, over the last years, there has been a rapid development of microscale devices that use microfluidic technology to create stable gradients compatible with real-time assays for cells, lately also in a three-dimensional setting, but there is currently no assay which integrates complex cell systems with the formation of stable gradients, live imaging and reversible system assembly.

A microfluidic multi-compartment device for neuroscience research has been developed [1]. The device comprises a poly(dimethylsiloxane) (PDMS) body defining two chambers, separated by a barrier to form fluidically isolated areas. A tissue culture dish is prepared to form a pattern of poly-L-lysine to guide attachment and growth of neurons to these dedicated areas, which become aligned with the chambers of the PDMS body when it is irreversibly bonded to the tissue culture dish. A microfluidic gradient is created by a series of microfluidic channels based on a controlled mixing of laminar flows. The gradient flow is then guided into the chambers.

Microfluidic devices for 3D cell cultures are known [2, 3]. Frisk et al. [2] discloses such a microfluidic device made of a silicon wafer, which was patterned through a photoresist and mask lithography to form fluid channels and a culture chamber on one side and inlets, outlets and filling holes on the other side. Vickerman et al. [3] discloses a microfluidic device made of a PDMS body comprising a culture chamber flanked by parallel fluid channels. In these two prior art solution, the culture chamber and the fluid channels are sealed by bonding the PDMS or silicon body to a glass cover. The culture chambers of these devices comprise an array of micro-pillars in order to provide support of a culture hydrogel matrix injected into the culture chamber.

### SUMMARY

The microfluidic devices of the prior art have limitations in their design, which requires micro-pillars in order to capture the culture hydrogel matrix and keep it in the culture chamber. However, even with the micro-pillar array, a dedicated microscope-based gel injection system must be used in order to inject the hydrogel into the chamber in order to prevent leakage thereof into the fluid channels. Additionally, enzymatic treatment may be needed in order to remove hydrogel plugs from the fluid channels.

Embodiments as disclosed herein solve these and other problems of the state of the art.

It is an objective to provide a fluidic culture device that can be used to conduct tests with cells, biological tissue, organs and/or small organisms.

It is a particular objective to provide a fluidic culture device capable of generating molecular gradients in 3D cultures.

These and other objectives are met by embodiments as defined by the accompanying patent claims.

Briefly, a fluidic culture device comprises a substrate made of a transparent polymeric material. The substrate comprises an open culture chamber in the form of a hollow in a bottom surface of the substrate. The culture chamber is provided for housing a culture matrix comprising a biological sample to be investigated. The open culture chamber is flanked on either side by fluid channels running from respective inlets in a top or end surface of the substrate to respective outlets or a common outlet in the top or an end surface of the substrate. Portions of the fluid channels are directly adjacent and in fluid contact with the culture chamber. According to the invention, prior adding the culture matrix to the culture chamber, removable channel plugs are removably arranged in the portions of the fluid channels adjacent the culture chamber. These channel plugs function as channel protecting devices that prevent any culture matrix material from entering the fluid channels and thereby obstruct or at least restrict the passage of a fluid, such as a solution, through the fluid channels.

When assembling a functional culture system, a liquid gel suspension is poured into the open culture chamber and is allowed to polymerize to form the culture matrix. The biological sample is added to the gel/matrix prior, during or after complete polymerization of the gel. The channel plugs present in the open fluid channels effectively prevent any leakage of the gel into the fluid channels.

The biological sample in the culture matrix present in the culture chamber may optionally be cultured in this open configuration of the fluidic culture device before removing the channel plugs and reversibly attaching the bottom surface of the substrate to a transparent cover disk. In the formed closed configuration, the cover disk encloses the culture chamber and the culture matrix therein and additionally functions as an enclosure to the fluid channels, which in the open configuration where open. Thus, the cover disk will prevent any leakage of fluid out from the fluid channels.

The culture system is then generally turned upside down so that the cover disk constitutes the bottom of the culture system. Fluid reservoirs are connected to the channel inlets to allow entry of the fluid into the fluid channels of the culture system. If the fluid entering the first fluid channel has a different concentration with regard to at least one substance than the fluid entering the second fluid channel, a gradient will be established over the culture chamber and the response of the biological sample to the gradient can be monitored.

Aspects of the invention include a fluidic culture device, a method of producing a fluidic culture device, a culture system and a culturing method in such culture system.

It has been shown herein that the fluidic culture device can be used to create growth factor gradients that induce directional angiogenesis in embryonic mouse kidneys and in clusters of differentiating stem cells. These results demonstrate that the fluidic culture device can be used to accurately manipulate complex morphogenetic processes with a high degree of experimental control.

### BRIEF DESCRIPTION OF THE DRAWINGS

The invention, together with further objects and advantages thereof, may best be understood by making reference to the following description taken together with the accompanying drawings, in which:
Fig. 1 is a drawing of a master that can be used to manufacture a fluidic culture device according to an embodiment;
Figs. 2A to 2D are schematics showing the assembly of a culture system based on a fluidic culture device according to an embodiment;
Fig. 3 is an illustration of a culture system based on a fluidic culture device according to an embodiment;
Fig. 4 is an upper view of a fluidic culture device equipped with a cover sheet facilitating tube attachment;
Fig. 5 is a cross-sectional view of a part of the fluidic culture device illustrated in Fig. 4;
Fig. 6 is an illustration of a fluidic culture device according to another embodiment;
Fig. 7 is an illustration of a fluidic culture device according to a further embodiment;
Fig. 8 is an illustration of a fluidic culture device according to yet another embodiment;
Fig. 9 is an illustration of a fluidic culture device according to still another embodiment;
Fig. 10 illustrates a time-lapse series of the central part of the culture chamber as a gradient of F-VEGFA is established;
Fig.11 illustrates a time-lapse series of F-VEGFA profiles as the gradient is established;
Fig. 12 is an illustration of the stable F-VEGFA profile across the full width of the culture chamber;
Fig. 13 illustrates a time-lapse series of the central part of the culture chamber as a gradient of FITC-dextran is established;
Fig. 14 illustrates FITC-dextran profiles recorded at the center of the culture chamber;
Fig. 15 is an illustration of the stable FITC-dextran profile across the full width of the culture chamber;
Fig. 16A illustrates embryonic E13.5 kidney stimulated with a growth factor gradient containing VEGFA, FGF2 and VEGFC for 48 hours;
Fig. 16B is a close-up of sprouts indicated by the box in Fig. 16A showing tip cells with characteristic protrusions;
Fig. 16C illustrated E13.5 kidney stimulated for 48 hours with a VEGFA gradient;
Fig. 16D illustrates control kidney growth for 48 hours in the absence of growth factors;
Fig. 17A illustrates embryoid bodies at day 8 prior to gradient stimulation;
Fig. 17B illustrates the same embryoid bodies as shown in Fig. 17A at day 9 after 25 hours of exposure to a gradient of VEGFA;
Fig. 18 is a gradient versus time graph showing the formation of a FITC-dextran gradient with a kidney positioned in the culture chamber;
Fig. 19 is a diagram illustrating the relationship between detected fluorescence and FITC-dextran concentration;
Fig. 20 is a flow diagram illustrating a method of manufacturing a fluidic culture device according to an embodiment; and
Fig. 21 is a flow diagram illustrating a culturing method according to an embodiment.

### DETAILED DESCRIPTION

Throughout the drawings, the same reference numbers are used for similar or corresponding elements.

The present invention generally relates to a fluidic culture device that can be used in connection with cell, tissue and/or organ culture experiments, and in particular such a culture device capable of generating molecular gradients in 3-dimensional (3D) cultures.

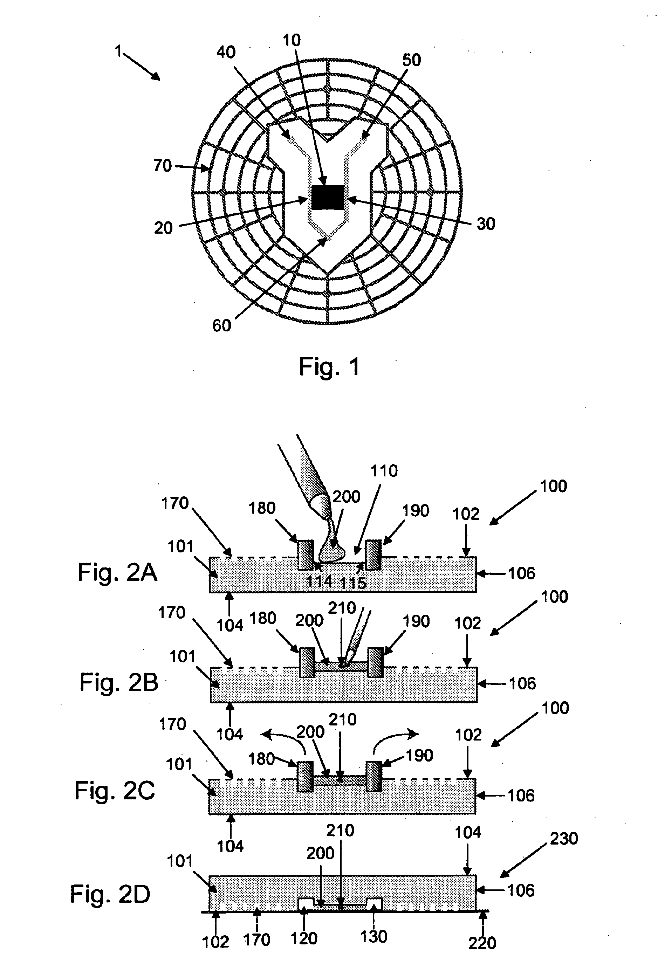

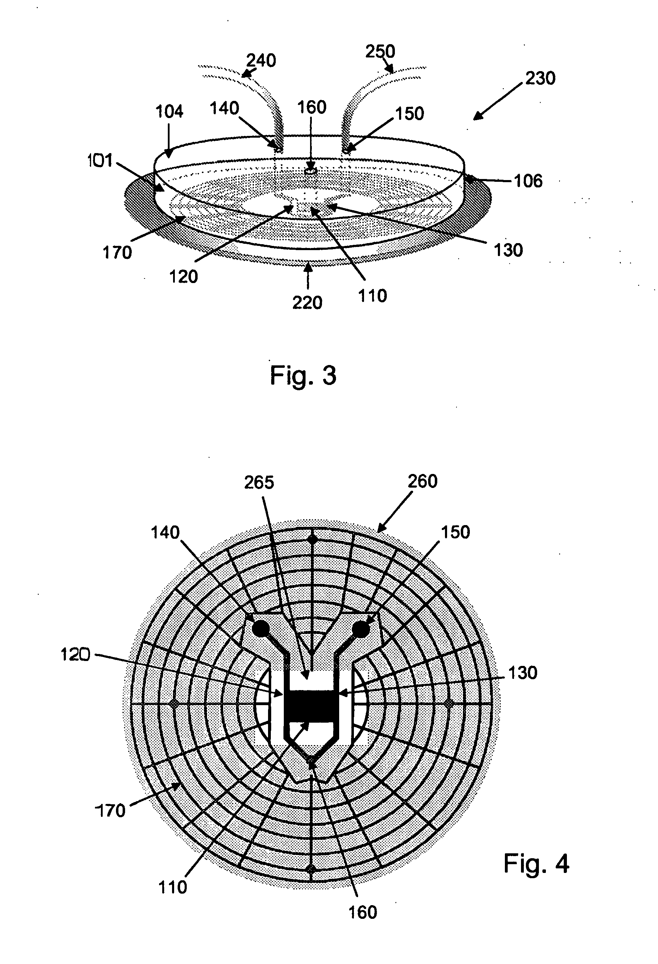

An aspect of the invention relates to a fluidic culture device 100, an embodiment of which is schematically illustrated in Fig. 3. The fluidic culture device 100 comprises a substrate 101 made of a transparent polymeric material. The substrate 101 can be of any shape and the circular design as shown in Fig. 3 is merely given as an illustrative but non-limiting example. Thus, other possible designs include, but are not limited, to rectangular, quadratic, triangular and oval overall shapes of the substrate 101. The substrate 101 comprises a top surface 104, an opposite bottom surface 102, which is more clearly seen in Figs. 2A to 2D, and at least one end surface 106 interconnecting the top surface 104 and the bottom surface 102. In Fig. 3, the substrate 101 merely comprises a single end surface 106 constituting the lateral surface of the cylindrical fluidic culture device 100. For other substrate cross-sectional shapes, three or more such end surfaces 106 may be present.

The fluidic culture device 100 has, as is seen in the cross-sectional views of Figs. 2A to 2D, an open culture chamber 110 present in a hollow in the bottom surface 102. The hollow may be in the form of a recess or indentation in the bottom surface 102, which is clearly seen in Fig. 2A. The overall shape of the open culture chamber 110 may vary in different embodiments as is discussed further below.

The open culture chamber 110 is flanked by and in fluid connection with a first fluid channel 120 on its first side 114 and by a second fluid channel 130 on a second, opposite side 116, see in particular Fig. 2D. Each of the fluid channels 120, 130 has a respective inlet 140, 150 and an outlet 160. The outlet 160 can be a common outlet as is illustrated in Fig. 3 or each fluidic channel 120, 130 can have a dedicated outlet 160. The inlets 140, 150 and outlet(s) 160 are provided in the top surface 104 or in an end surface 106 of the substrate. In a particular embodiment as illustrated in Fig. 3, the inlets 140, 150 and outlet(s) 160 are all provided in the top surface 104 of the substrate 101. However, if the substrate 101 is thick enough the inlets 140, 150 can be provided in one or two end surfaces 106 and the outlet(s) 160 is(are) provided in one or two end surfaces 106, preferably opposite end surfaces or the same end surface 106, depending on the particular substrate design.

The fluidic channels 120,130 are used, during operation of the fluidic culture device 100, for carrying a fluid that enters the culture device 100 at the inlets 140, 150 and leaves the culture device 100 at the outlet(s) 160. The fluid is in particular a solution or liquid fluid that advantageously contains at least one test substance, if the fluidic culture device 100 is employed for investigating the response of a biological sample present in the culture chamber 110 to the at least one test substance. Alternatively, the fluid can be in the form of a gas flow that is lead into the fluid channels. 120, 130 or a liquid fluid with dissolved gas therein. In an embodiment, the fluid entering the inlet 140 of the first fluid channel 120 is identical to the fluid entering the inlet 150 of the second fluid channel 130. However, the fluidic culture device 100 is particularly suitable for establishing a gradient of at least one test substance over the culture chamber 110. In such a case, the fluid entering the first fluid channel 120 comprises the at least one test substance at a first concentration, whereas the second fluid channel 130 carries a fluid having the at least one test substance at a second concentration (possibly zero concentration) that is different from the first concentration.

The portions of the fluid channels 120; 130 adjacent the open culture chamber 110 are preferably parallel. This is in particular advantageous when establishing a gradient of a substance over the culture chamber 110.

The fluidic culture device 100 additionally comprises respective removable channel plugs 180, 190 removably arranged in the first and second fluid channels 120, 130. In such a case, a first removable channel plug 180 is removably arranged in a portion of the first fluid channel 120 adjacent the first side 114 of the open culture chamber 110. The second removable channel plug 190 is similarly removably arranged in a portion of the second fluid channel 130 adjacent the second, opposite side 116 of the culture chamber 110.

The removable channel plugs 180,190 of the fluidic culture device 100 achieve several advantageous effects. Firstly, the channel plugs 180, 190 occupy the portions of the fluid channels 120, 130 that are immediately adjacent the open culture chamber 110. This means that the plugs temporarily isolate the fluid channels 120, 130 from the culture chamber 110, which is important when introducing a culture matrix 200 in the open culture chamber 110. The channel plugs 180,190 effectively prevent the culture matrix 200 from unintentionally entering the fluid channel 120, 130 and thereby prevent the fluid channels 120, 130 from becoming fully or at least partly obstructed or blocked by culture matrix material. This is a major problem in the prior art fluidic culture devices, such as disclosed in documents [2, 3]. No complex, high-precision culture matrix delivering system nor any separate process step of enzymatically degrading any culture matrix material entering the fluid channels 120, 130 is therefore needed according to the invention.

Additionally, the removable channel plugs 180, 190 help to define the culture chamber limitations during the formation of the culture matrix 200 in the open culture chamber 110. Thus, the two channel plugs 180, 190 constitute opposite wall limitations for the open chamber device 110 during the culture matrix formation step. This allows the correct formation of the culture matrix 200 within the boundaries of the open culture chamber 110 and not any leakage out into the fluidic channels 120, 130.

The removable channel plugs 180, 190 also achieve advantageous effects if the fluidic culture device 100 is employed for cell culturing without any culture matrix. In such a case, the removable channel plugs 180, 190 prevent, when adding medium including cells of interest to the open culture chamber 110, the cells from leaving the culture chamber 110 and unintentionally entering the fluid channels 120, 130. After culturing the cells for a period of time, they become attached to the bottom surface of the open culture chamber 110. At this point the removable channel plugs 180, 190 can be safely removed since the cells will be fixed through the cell-to-surface attachment to the culture chamber surface.

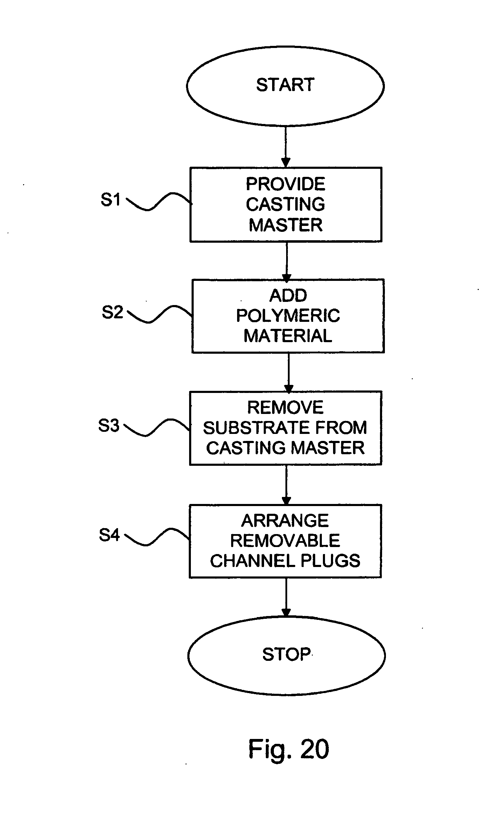

The fluidic culture device 100 is advantageously manufactured in a casting procedure according to another aspect of the invention. Such a production method is schematically illustrated by the flow diagram of Fig. 20. The method starts in step S1, which involves providing a casting master 1, see Fig. 1, having a chamber defining structure 10 flanked on either sides of respective channel defining structures 20, 30.

The channel defining structures 20, 30 run from respective start positions 40, 50 corresponding to the positions of the inlets 140,150 in the final fluidic culture device 100 and to a common or respective end positions 60 corresponding to the position(s) 160 of the outlet(s) in the fluidic culture device 100.

The casting master 1 can be fabricated in, for instance, SU-8 resist, which is an epoxy-based negative photoresist that is available from MicroChem Corp., Newton, MA, USA. In an embodiment of step S1, the SU-8 resist is fabricated in three layers with different heights corresponding to the channel defining structures 20, 30, the chamber defining structure 10 and optional but preferred vacuum channel defining structures 70. Structures in the first and third layers can be fabricated using standard SU-8 processing, whereas the second and thickest layer is preferably processed similar to the methods described by Lin et al. [4], which is hereby incorporated by reference for the purpose of disclosing how to form a layer of the SU-8 resist. The SU-8 photoresist can be applied onto a wafer, preferably slightly heated in order to facilitate even distribution of the thick layer, and a scraper can be used to spread the SU-8 material over the substrate. The wafer is then baked followed by lithography processing.

Thereafter and once the third layer of the casting master 1 has been formed, it can be hard-baked to form the final casting master 1.

A next step S2 adds a transparent polymeric material to the casting master 1 and allows the polymeric material to polymerize to form the substrate 101. The formed substrate 101 is then removed in step S3 from the casting master 1 and preferably sterilized, such as in 70 % ethanol. Holes for the inlets 140, 150 and outlet(s) 160 can be made in the top surface 104 or in the end surface 106 to get access to the respective ends of the fluid channels 120,130 preferably present as open channels or recesses in the bottom surface 102. The removable channel plugs 180, 190 are then removable arranged in the fluid channels 120,130 in step S4 as previously described to be present adjacent the open culture chamber 110 and temporarily block the fluid channels 120, 130 from entry of any culture matrix 200 to be entered in the culture chamber 110.

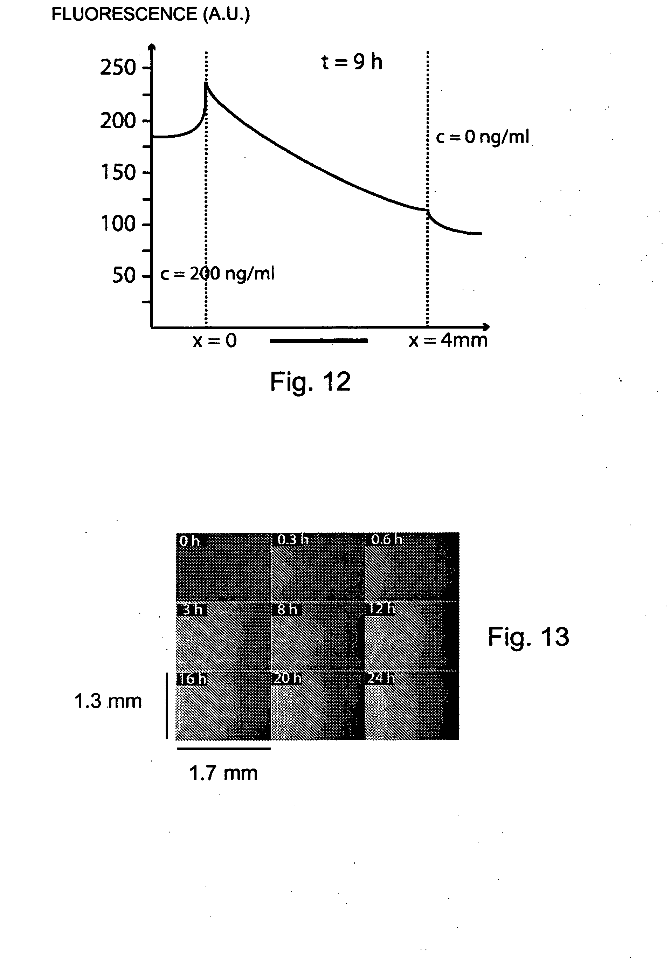

In operation, the fluidic culture device 100 and the substrate 101 forms part of a culture system 230 as illustrated in Fig. 3. In the culture system 230, the substrate 101 is reversibly bound to a transparent cover disk 220 to thereby enclose the culture chamber 110, the culture matrix therein 200 and the fluid channels 120,130. Figs. 2A to 2D and 13 schematically illustrate assembling of the culture system 230 and operation thereof in a culturing method according to a further aspect of the invention.

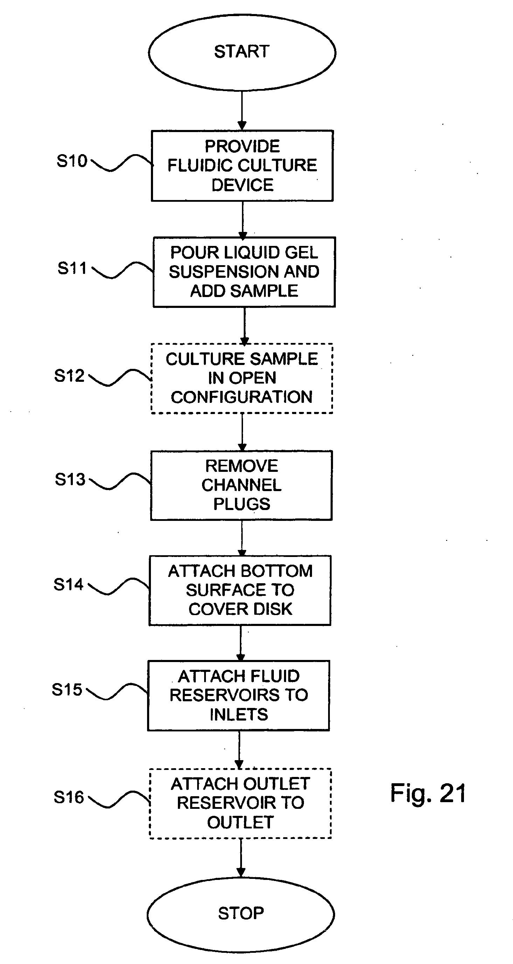

Fig. 2A illustrates a cross-sectional view of a part of the substrate 101 over the portion comprising the open culture chamber 110. The assembling and culturing method starts in step S10 of Fig. 21, in which the fluidic culture device 100 of the invention is provided as previously described and a cell matrix 200 is formed in step S11 in the culture chamber 110. The cell matrix 200 is preferably formed by pouring a liquid gel suspension into the open culture chamber 110 and allowing the gel to polymerize to form the culture matrix 200. The present invention can be used in connection with any type of culture matrix material known in the art and that can be poured into the culture chamber 110 and polymerize to form a solid culture matrix 200. Additionally, the culture matrix 200 once formed should preferably be transparent to allow visual inspection and visual access to the biological sample 210 to be included therein.

Examples of suitable matrix material include collagen materials, such as collagen I. Collagen I is well documented to support 3D cultures. Other gels that can be used include ECM gels, such as Matrigel (BD Bioscience, Bedford, MA, USA) or hydrogels, including a mixture of phenylalanine (Phe) dipeptide formed by solid-phase synthesis with a fluorenylmethoxycarbonyl (Fmoc) protector group on the N-terminus, and Fmoc-protected lysine (Lys) or solely phenylalanine. However, any type of biocompatible matrix could be used, given the martix can be applied in soluble form and cast or polymerized to form a solid culture matrix 200.

The gel suspension can be applied in a single layer or in multiple, i.e. at least two, layers to form different cell matrices and/or cell systems.

In a preferred embodiment, the culture matrix 200 occupies the full area and volume of the culture chamber 110. This means that opposite end sides of the culture matrix 200 is in connection with the removable channel plugs 180, 190 and, when these channel plugs 180, 190 are removed, with the adjacent fluid channels 120,130.

As is seen in Fig. 2A, the removable channel plugs 180, 190 of the invention effectively prevent the liquid gel suspension from entering the fluid channels 180, 190 and thereby prevent the culture matrix material from unintentionally blocking or obstructing the fluid channels 180, 190 during the assembly process. No high-precision microscope based tool is needed to apply the gel suspension and a simple pipette loaded with the gel suspension can be used due to the protective effect of the channel plugs 180. 190. Additionally, no extra channel cleaning step using an enzymatic solution with the purpose of dissolving or disintegrating any culture matrix plugs formed in the fluid channels 180, 190 is needed, thereby significantly simplifying the assembly process.

A biological sample 210 is added to culture matrix 200 in step S11, which is schematically illustrated in Fig. 28. The addition of the sample 210 can be performed once the polymerization has been completed to form the solid culture matrix 200. Alternatively, the sample 210 is added to the gel during the polymerization or indeed before the polymerization of the gel suspension has started.

It is a unique feature that the biological sample 210 can be precisely positioned in the culture chamber 110 during or after the polymerization step. This can be done by either making the culture matrix 200 in several layers or steps so that the biological sample 210 is positioned in a desired layer with a defined distance from the top or the bottom of the culture chamber 110. Producing the culture matrix 200 within the fluidic culture device 100 in an open configuration makes it possible to make multiple matrix layers in the culture chamber 110, and at the same time to seed different cells and biological samples in different layers. This is technically very challenging in closed systems. Also, the biological sample 210 can be injected using a stabilized syringe injector at a defined location in already polymerized matrix as is shown in Fig. 2B. Alternatively, a Pasteur pipette can be used for sample introduction. Positioning of cells, organs or model organisms in liquid matrix can be done using a thin stick to push around the material. Thus, taken together, biological samples 210 can be precisely positioned in three dimensions during, or in the case of dissociated cells during or after, completed matrix polymerization in the culture chamber 110. Notably, any type of biocompatible matrix 200 can be used that can be applied in soluble form and then be made to polymerize to give rise to a gel.

The biological sample 210 introduced in the culture matrix 200 can be any type of biological material that one wants to investigate. Non-limiting example encompass all types of cells, including clusters of cells and stem cells, embryonic organs, small model organisms, such as zebrafish and nematodes. Actually, all sorts of cells or pieces of tissue isolated from experimental animals and patients such as tumor cells, biopsies, endothelial cells, nerve cells etc. can be precisely positioned in the culture chamber 110 during the polymerization step.

The size of the culture chamber 110 of the substrate 101 can be designed and changed to fit the specific experimental needs. For instance, investigating larger tissue samples, organs and even complete organisms generally requires larger culture chamber volumes as compared to individual cells, cell clusters and other smaller biological samples. The culture chamber 110 can consequently be designed to be compatible with cultures of relatively large cell clusters, embryonic organs or small experimental organisms, and its dimensions may be in the millimeter scale and even up to centimeter scale. An advantage of the invention is that the size of the culture chamber 110 is also compatible with growth of small model organisms, such as zebrafish embryos, and the culture chamber dimensions can easily be changed to fit specific experimental needs. Further, the culture chamber 110 is well suited for studies of interactions between different cell types as cells can be precisely positioned in the culture chamber 110. The size of the culture chamber 110 and also of the fluidic culture device 100 can easily be changed by changing the size and geometry of the casting master 1 used for producing the substrate 101 of the fluidic culture device 100.

Once the biological sample 210 has been introduced in the culture matrix 200 in step S11, the biological sample 210 may optionally be cultured in the open configuration of the fluidic culture device 100 in step S12. Open configuration indicates that one has full access to the culture chamber 110 and the culture matrix 200 with the biological sample 210 and no cover disk has yet been attached to the substrate 101.

This is a unique feature that cells and other biological samples 210 after precise positioning in the three-dimensional culture matrix 200, as described above, can be grown in what is referred to as the "open configuration" with the culture chamber 110 facing upwards as shown in Fig. 2C. Culturing with the fluidic culture device 100 in the open configuration is enabled by adding cell medium to the top of the culture chamber 110. Replacing the medium at regular intervals allows for prolonged culture, for in principle as long as required, normally several days or weeks. Growth in the open state makes it possible to follow cells and other biological samples 210 with live imaging prior to system assembly, and to make manipulations of the biological sample 210 if required during this time, such as changing growth conditions or to for example add in more cells.

The next step S13, which may be conducted prior, during or after the optional culturing step S12, removes the channel plugs 180,190 from the fluidic channels 120,130. This is schematically illustrated in Fig. 2C. The fluidic culture device 100 with the culture matrix 200 and biological sample 210 is now ready for attachment to a transparent cover disk to form a closed configuration in which the culture chamber 100 and the culture matrix 200 therein are fully enclosed in the resulting culture system 230.

The attachment step S14 involves reversibly attaching the bottom surface 102 of the substrate 101 to the transparent cover disk 220 to thereby enclose the culture chamber 110 and the culture matrix 200. Additionally, the cover disk 220 also prevents leakage of any fluid to be introduced in the fluid channels 120, 130. Thus, the cover disk 220 preferably forms the bottom enclosure of the fluid channels 120, 130 once the assembled culture system 230 has been turned upside down as illustrated in Fig. 2D.

The attachment of the bottom surface 102 of the substrate 101 to the cover disk 220 is a reversible attachment implying that the cover disk 220 can later on be removed and detached from the fluidic culture device 100 without any significant damages to the substrate 101 or the culture matrix 200. Thus, the substrate 101 does not become permanently bound to the cover disk 220 but instead provides a fluid-tight connection therebetween that can be reversed by carefully pulling the substrate 101 away from the cover disk 220.

The reversible attachment is achieved by the creation of suction pressure between the cover disk 220 and the bottom surface 102. This suction pressure is enough to firmly attach the substrate 101 to the cover disk 220 and prevent any fluid leakages out from the fluid channels 120, 130. In a preferred embodiment, the suction pressure is enhanced by a network of so-called vacuum channel 170 circumferentially provided in the bottom surface 102 relative the open culture chamber 110. In such a case, the casting master 1 used for forming the substrate 101 comprises vacuum channel defining structures 70 as illustrated in Fig. 1 to form a network of such channels 170 provided around and away from the preferably central portion of the substrate 101 containing the culture chamber 110 and the fluid channels 120, 130.

The vacuum channels are open channels 170 present in the bottom surface 102 and can be in the form of recesses therein. The number of such interconnected channels 170 and their position in the substrate 101 can vary according to different embodiments. It is though preferred to have vacuum channels 170 encircling the culture chamber 110 and the fluid channels 120, 130 as is seen in Fig. 3. Additionally, it is preferred if the innermost vacuum channels 170 are distanced a bit away from the fluid channels 120, 130 to prevent unintentionally leakage of the fluid from the fluid channels 120, 130 into the network of vacuum channels 170.

The transparent cover disk 230 can be any type of flat surface that is at least transparent in the portion of the disk that is aligned with the culture chamber 110 to allow visual access to the biological sample 210 in the culture system 230. Thus, any remaining part of the cover disk 230 can indeed be transparent but does not have to be it as long as the chamber-aligned portion is transparent.

The cover disk 220 can for instance be a Petri dish or a glass plate. The culture system 230 is assembled with the help of vacuum channels 170 that run along the perimeter of the substrate 101. The vacuum channels 170 may be connected to an external vacuum source (not illustrated) in order to provide additional suction pressure. However, generally gently pressing the substrate 101 against the flat surface will create enough adherence to the surface without the need for any external vacuum source, though external vacuum may be applied to the vacuum channels 170 to ensure stronger assembly. The culture chamber 110 where biological material 210 is grown is generally positioned in the middle of the substrate 101, and this is the first time that a culture chamber 110 for sample culture in a three-dimensional matrix 200 in the millimeter scale or even up to centimeter scale is combined with a system for reversible assembly.

Uniquely, as the culture system 230 is made in one substrate piece, it can easily be turned up side down with the culture chamber 110 containing the three-dimensional polymerized matrix 200 and integrated precisely positioned biological sample 210, to be attached to a specific location at any type of flat surface with the help of the suction power developed by the vacuum channels 170. The fact that the fluidic culture device 100 is made as one piece gives a great advantage over devices that require multipart assembly. The assembly of the culture system 230 not only creates the sealed culture chamber 110 with a culture matrix 200 but also results in the formation of the fluid channels 120, 130 where one side of the fluid channels 120,130 will face the culture matrix 110.

A next step S15 attaches a respective fluid reservoir 240, 250 to the inlets 140,150 provided in the top surface 104 of the substrate 101 to thereby allow a fluid from the reservoir to enter the fluid channels 120, 130. A corresponding outlet reservoir (not illustrated) is optionally attached in step S16 to the outlet 160 to allow the fluid from running from the fluid reservoirs 240, 250 through the fluid channels 120, 130 and out into the outlet reservoir. The culture system 230 of the invention is now fully operational and can be used for establishing a gradient of at least one substance over the culture chamber 110 and the culture matrix 200 by providing different concentrations of the at least one substance in the fluid entering the first inlet 140 as compared to the fluid entering the second inlet 150.

Respective pumps or other fluid pushing equipment can be used for moving the fluid from the fluid reservoirs 240, 250 into the fluid channels 120, 130 and out from the outlet 170 and optionally into the at least one outlet reservoir.

The culture system 230 described herein and illustrated in Fig. 3 enables formation of controllable gradients in 3D culture systems over long periods of time, enabling the study of complex multi-step morphogenetic processes. The culture system 230 can be used to create molecular gradients to affect and direct complex biological processes in biological material such as cells including clusters of cells, stem cells, embryonic organs, small model organisms such as zebrafish and nematodes, and all sorts of cells isolated from experimental animals and patients such as tumor cells, endothelial cells, nerve cells, differentiating stem cells, small mouse embryonic organs, zebrafish embryos etc. Non-limifing biological processes that can be monitored by the culture system 230 include cell migration, cell differentiation, cell proliferation and survival.

Importantly, the culture system 230 is compatible with live imaging of the biological sample 210 in the culture chamber 110, and advanced microscopy may therefore be applied to perform e.g. real time photo-manipulation of biological samples 210. The culture system 230 has the potential to advance translational research primarily in the fields of developmental biology and experimental medicine. Here, 3D tissue culture models providing a high degree of experimental control are often necessary for reductionist approaches to decipher detailed molecular mechanisms.

Thus, the culture system 230 can be used to investigate, even in real time, the response to the biological sample 210 to the gradient of at least one substance established between the two fluid channels 120, 130 and over the culture matrix 200. The gradient over the culture matrix 200 in the culture chamber 110 is formed by diffusion of the at least one substance from one of the fluid channels 120 to the other fluid channel 130. Thus, the diffusion is from a so-called source channel, which has a higher concentration of the at least one substance in the fluid relative the other fluid channel, denoted sink channel. In a preferred embodiment, the flow rates of the fluid in the two fluid channels 120,130 is preferably kept substantially similar since then no flow of the fluid is recorded through the culture chamber 110. Substantially similar indicates that the two flow rates are preferably identical but can differ slightly due to inherent variations in the flow rate of the pumping systems. Thus, the difference in flow rate in the two fluid channels 120, 130 are preferably less than 10 %, more preferably less than 5 %, such as less than 2.5 % and most preferably less than 1 %. If there is no net flow of the fluid over the culture chamber 110 and the parts of the fluid channels 120,130 adjacent a culture chamber 110 with rectangular or quadratic bottom area, the gradient over the culture chamber 110 will be linear.

However, variously shaped gradients can be generated over the culture chamber 110 by setting the flow in the source and the sink channels 120, 130 at different rates. Different flow rates in the source and the sink channels 120, 130 can also be used to create flow in the culture chamber 110. The directionality of the flow will be from the fluid channel with the highest flow rate towards the fluid channel the lower flow rate. The effects of flow and shear stress created by the flow on cells can thus be studied in the culture system 230, and this is of great interest as shear stress has been shown to impact various cell processes, such as angiogenesis and remodeling of vascular networks.

All types of biological samples 210 with a sample size smaller than the culture chamber 110 can be grown in the culture system 230 in its closed state for in principle as long as wanted. This is made possible by that the culture system 230 is insensitive to perturbations of flow, and syringes pumping media to the sink and source channels 120, 130, thus constantly feeding nutrients and oxygen to the culture chamber 110, may be refilled for as many times as required during the course of an experiment, also without noticeably affecting the gradient shape. It is possible to perform live-imaging during the full course of an experiment, including replacement of syringes.

The culture system 230 can after being assembled to the closed state be dissembled or opened to again grow biological samples in the open state. This is possible due to the reversible attachment of the bottom surface 102 to the cover disk 220. The culture system 230 can then also be closed again if desired. This culture system 230 can in theory be closed and opened for as many times as required.

After a completed experiment, the intact biological sample(s) 210 can be retrieved from the culture chamber 110 using, for example, forceps and/or scalpel once the substrate 101 has been removed from the cover disk 220. The sample 210 can then be subject to any type of analysis. For example, fixation and immunohistochemical analysis of the biological sample 210, dissociation of the gel and isolation of material for cell sorting, such as fluorescence-activated cell sorting (FACS), isolation of cells and mRNA for PCR, isolation of cells and proteins for western blotting, etc.

The biological sample 210 can also be retrieved from the culture chamber 110 for further culture and experimentation in other cell culture systems, or for injection into laboratory animals for further experimentation.

The substrate 101 in the fluidic culture device 100 is made of a transparent polymeric material. Transparent polymeric material allows optical investigation of the biological sample 210 in the culture matrix 200 by, for instance, microscopy. The polymeric material is preferably elastic to enhance the reversible attachment through a generated suction pressure to the cover disk 220. A further preferred feature of the polymeric material is permeability to gas. In such a case, oxygen needed for the viability of the biological sample 210 in the culture matrix 200 can enter the culture chamber 110 through all sides of the substrate 101. Additionally, any gases produced by the biological sample 210 can exit the culture chamber 110 through the substrate material. Though, gas permeability is preferred, it could be possible to use gas impermeable polymeric materials, such as thermoplastic materials. In such a case, any gas transportation to and from the biological sample 210 is conducted through the fluid channels 120, 130. This, however, generally reduces the number of cells that the biological sample 210 consists of as otherwise cell death occurs. It is also hard to use the gas impermeable substrate materials in connection with larger biological samples, such as tissue, organs or complete organisms unless larger fluid flows through the channels and/or larger dimensions are used to compensate for the lack of gas impermeability. An alternative could in this case be to use a gas permeable cover disk 220 attached to the substrate 101 during system assembly.

There was no evidence of cell death in cultures of cells or organs grown in the fluidic culture device 100 for 48 hours, demonstrating that the cells in the fluidic culture device 100 receives sufficient levels of oxygen and nutrients with a fluidic culture device produced in gas permeable polymeric material.

A preferred polymeric material that meets the above-listed preferred features include poly(dimethylsiloxane) (PDMS).

The fluid reservoirs 240, 250 illustrated in Fig. 3 have been exemplified by tubes connected to respective fluid sources (not illustrated). These tubes can be attached to the substrate 101 by pushing them into the inlets 140, 150 and optionally push an outlet tube into the outlet 160. In such a case, the outer diameter of the tubes is preferably slightly larger than the inner diameter of the inlets 140, 150. and outlet 160 to form a fluid tight connection.

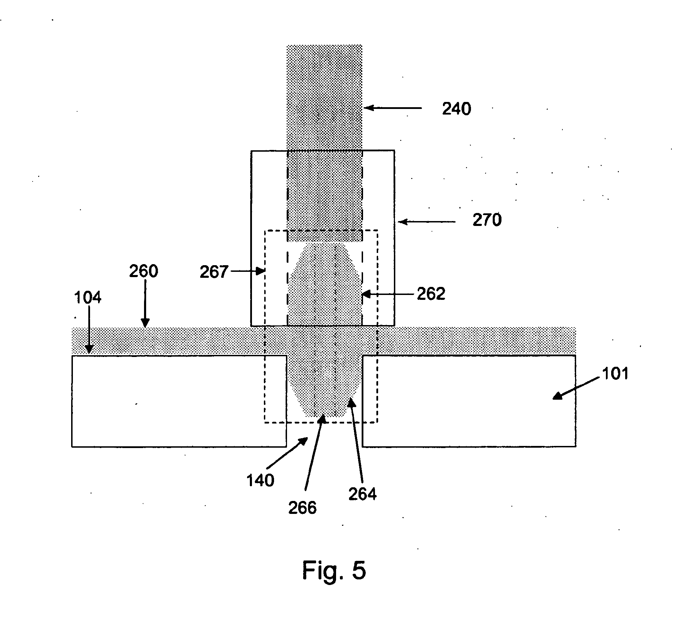

Figs. 4 and 5 illustrate an alternative embodiment of attaching tubes to the inlets 140, 150 and outlet 160. Fig. 4 illustrates an upper view of the fluidic culture device 100, whereas Fig. 5 is a cross-sectional view of a portion of the substrate taken over one of the inlets 140. In this embodiment, a cover 260 in the form of sheath or disk is attached to the top surface 104 of the substrate 101. This attachment of the cover 260 can be a reversible attachment as between the bottom surface 102 and the transparent cover disk 220. In such a case, the cover 260 is simply pushed tightly against the top surface 104 to form suction pressure that reversibly binds the cover 260 to the substrate 101. However, as the culture chamber 110 is not accessible from the top surface 104, the cover 260 could alternatively be irreversibly attached to the top surface 104.

Such irreversible attachment can be performed through surface modification of the top surface 104 by exposure to air plasma to render the top surface 104 hydrophilic. The oxidized top surface then binds irreversibly to the cover 260, in particular if the substrate 101 is made of PDMS and the cover is a plastic or glass material. Other types of irreversible binding include various glues that can be used between polymeric material or between a polymeric material and glass.

The cover 260 can advantageously be transparent or at least comprise a transparent window 265 aligned with the culture chamber 110 to allow optical investigation of the biological sample 210 therein.

The cover 260 also comprises, which is more clearly seen in Fig. 5, a connecting structure 267 that is aligned with the channel inlet 140. More preferably, the cover 260 comprises one such connecting structure 267 aligned with each inlet 140, 150 and optionally the outlet 160 of the fluidic culture device 100. The connecting structure 267 comprises a channel connecting part 264 extending from a bottom surface of the cover 260 into the inlet 140 (or an outlet) of the fluid channel. The channel connecting part 264, thus, protrudes into the inlet 140 (or outlet). A tube connecting part 262 is aligned with the channel connecting part 264 and extends from a top surface of the cover 260. A bore or channel 266 runs through the channel connecting part 264 and the tube connecting part 262 to allow access to the inlet 140 (or outlet).

The tube connecting part 262 is then connected to the tube of the fluid reservoir 240 or outlet reservoir. An advantage of this structure is that the tube connecting part 262 and indeed the whole cover 260 can be made of a different material than the substrate 101. This means that the cover material can be selected to allow easy and safe connection of the tube connecting part 262 with the tube, for instance by welding or threading the tube over the tube connecting part 262, which is schematically illustrated by the reference number 270.

The cover material is advantageously a polymeric material, such as polystyrene (PS).

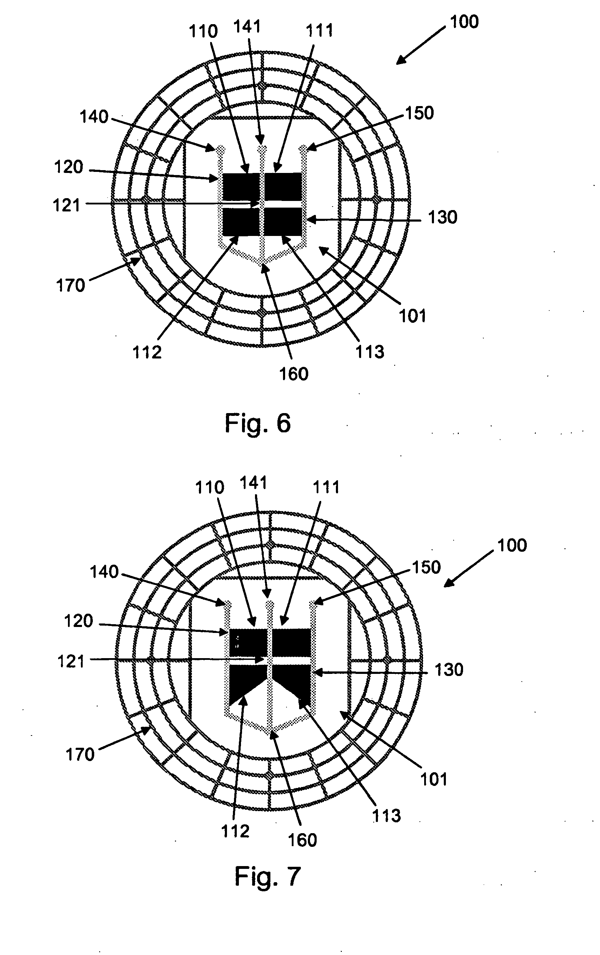

The fluidic culture device 100 disclosed in Figs. 2-4 and described above contains one culture chamber 110. The invention is, though, not limited thereto. Fig. 6 illustrates an upper view of another embodiment of a fluidic culture device 100. This fluidic culture device 100 illustrates the concept of having parallel and serial culture chambers 110-113. Thus, a first culture chamber 110 is provided in between a first fluid channel 120 and a second fluid channel 121. A second culture chamber 112 is also provided between these fluid channels 120, 121 but is provided downstream of the first culture chamber 110. In other words, the first culture chamber 110 is closer to the channel inlets 140, 141 as compared to the second culture chamber 112 that is closer to the outlet 160.

Serial culture chambers 110, 112 can be used to provide a first biological sample in the first culture chamber 110 that produces and secretes, when exposed to a gradient or an even level of at least one substance, at least one agent or molecule of interest. The biological sample provided in the second culture chamber 112 is then responsive to this agent or molecule of interest. This means that the fluidic culture device 100 can be used to investigate the production and secretion of molecules together with the response of cells and other biological material to such secreted agents or molecules. The interaction between different cell types can therefore be investigated with the fluidic culture device 100.

The fluidic culture device 100 of Fig. 5 also comprises a third culture chamber 111 and a fourth culture chamber 113 present in between the second fluid channel 121 and a third fluid channel 130. With such a setting it is possible to establish two different gradients, one over the first and second culture chambers 110, 112 and a second gradient over the third and fourth culture chambers 111, 113. This concept can of course be extending further to have more than two serial culture chambers and/or more than two parallel sets of culture chambers.

Using more than one culture chamber in the fluidic culture device additionally allows the provision of a control culture chamber within the same physical culture device as the culture chamber(s) containing the test biological sample and exposed to a test gradient.

The size and shape of the culture chamber(s) of the fluidic culture device can be used to create gradients of different shapes, i.e. also non-linear gradients, that will affect the biological sample differently. Thus, by varying the shape of the culture chamber, the steepness of the gradients can be affected.

Fig. 7 illustrates this concept by disclosing the fluidic culture device 100 of Fig. 6 but with the second and fourth culture chambers 112, 113 not having rectangular shape as seen from above. In dear contrast, in this case the area of the culture chambers 112, 113 facing one of the fluid channels 120, 130 is comparatively larger than the opposite area facing the opposite fluid channel 121. This will cause non-linear gradients in these culture chambers 112,113.

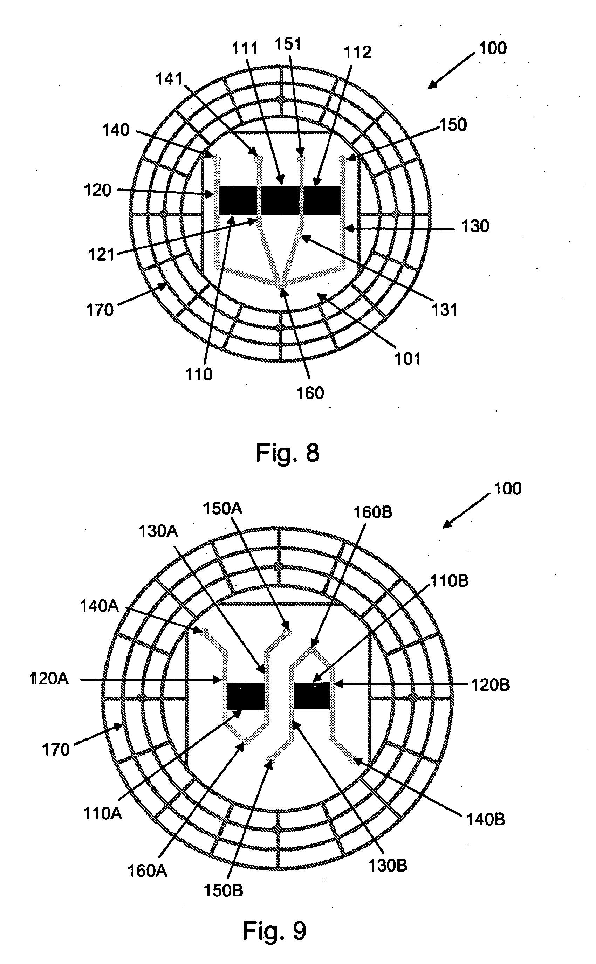

Fig. 8 schematically illustrates another embodiment of the fluidic culture device 100 by having more than three serial culture chambers 110, 111, 112 each provided between a respective pair of fluid channels 120, 121, 130, 131. With this setting three different gradients of a same substance or set of substances can be investigated by having four different concentrations of the at least one substance in the fluid entering the channel inlets 140, 141, 150, 151.

It is also possible to have multiple isolated culture chambers 110A, 110B in the same fluidic culture device 100 as is illustrated in Fig. 9. Each such culture chamber 110A, 110B is flanked by dedicated fluid channels 120A, 130A, 120B, 130B having separate inlets 140A, 150A, 140B, 150B and outlets 160A,160B.

The fluidic culture device of the invention can, thus, hold multiple isolated or serial or parallel culture chambers for parallel analysis of many sample and also investigating interaction between different biological samples. One and the same fluidic culture device can therefore be used to study multiple gradients, possibly of different shape and their simultaneous effects on the biological samples, including all types of cell behavior.

The different embodiments of the fluidic culture device discussed above and disclosed in Figs. 3, 4, 6-9 can be combined. For instance, the cover with connecting structures in Fig. 4 can be used in connection with any of the embodiments shown in Figs. 6-9. Usage of a culture chamber design to achieve non-linear gradient as illustrated in Fig. 7 can be applied to any of the embodiments in Figs. 3, 4, 6, 8 and 9.

Additional fluidic channels can be introduced in the fluidic culture device to create more complex gradient shapes, or beams of factors, chemical compounds, reagents and/or tracers, in the culture chamber or connected to the inlet or outlet channels, to enable better detection of biological processes.

The channel outlet(s) of the fluidic culture device can be connected to any type of online detector system, to detect factors, such as proteins produced by the biological sample in the culture chamber or to detect consumption of any type of molecule that can be detected by an on-line detector by the device. For example, changes in insulin production by islets of Langerhans grown in the fluidic culture device can be measured as a result of precise sample manipulations by measuring insulin in the fluid coming out from the culture chamber of the culture device.

As will become evident from the experiment section, it is shown herein that the fluidic culture device according to the present invention can be used to create growth factor gradients that induce directional angiogenesis in embryonic mouse kidneys and in clusters of differentiating stem cells. These results demonstrate that the fluidic culture device can be used to accurately manipulate complex morphogenetic processes with a high degree of experimental control.

Two examples of real-time assays for the study of directional angiogenesis, based on either the use of small embryonic mouse kidneys or clusters of differentiating mouse embryonic stem cells are presented in the present application. In these assays it is possible to study the formation of tip cells and regulation of vessel sprouting and branching as a response to graded stimulations. These are processes central to angiogenesis, and therefore represent potential targets for angiogenic therapy.

Taken together, the fluidic culture device described here can be used to induce directional angiogenesis and remodeling of the pre-existing vascular plexus in embryonic mouse kidneys. The fluidic culture device should when combined with material isolated from the full repertoire of transgenic animals facilitate studies of the precise role of genes involved in the regulation of sprout guidance and sprout organization. Examples of relevant genes to study in this context are genes belonging to the recently described delta-like 4 (DII4)-Notch1 signaling axis.

It is an interesting perspective that the fluidic culture device can be used to manipulate cultures of biological material of human origin where it for ethical reasons is impossible to perform corresponding experiments in vivo. Preclinical testing has so far relied on experimentation in various animal models. In the future, application of human embryonic stem cells may in some cases constitute an additional step for drug testing. The fluidic culture device and assay combined with human stem cells and the emerging technologies in this field also offers new possibilities for the study of basic mechanisms of human embryonic development.

Additionally, the fluidic culture device can be used to determine effective or lethal doses for any soluble compound that can be used to create a gradient or an even concentration in the fluidic culture device, by analyzing concentration-dependent effects on cells that are in the culture chamber. The fluidic culture device can thus be used to screen for the biological activities of pharmacological compounds or for the testing of new drugs.

The fluidic culture device is also well suited for studies of tumor cells, and tumor/cancer material isolated from patients. The effects of different compounds on cancer cells can be studied; drug screening, toxicity tests, early testing of new treatments on tumor cells, normal tissue and blood vessels.

The fluidic culture device of the invention can be used for monitoring numerous biological processes in relation to gradients in the fluidic culture device. Examples of such biological processes include: cell survival, cell division, cell death, cell migration, cell differentiation, cell communication, tissue organization, paracrine signaling in tissues between different cell types, and general or specific developmental processes in organs and whole model organisms. The fluidic culture device is especially well suited for studies of angiogenesis and nerve cell formation, communication and growth.

As stated above, the fluidic culture device can be used to control complex biological processes to facilitate research aimed at understanding fundamental cell function in biological material derived from any tissue in the human body or biological material from any type of animal experimental model, including mouse, rat, and zebrafish.

It is of particular interest that biological material of human origin, such as human stem cells or biopsies from normal or pathological human tissues, such as tumor tissue, inflamed tissue, bone marrow or blood, can be used in the culture system.

Preclinical testing has so far relied on experimentation in various animal models. In the future, application of human stem cells or other cell types of human origin, such as cancer cells, blood vessel cells, nerve cells, muscle cells, blood cells, etc., may constitute an additional step for (personalized) drug testing. The fluidic culture device and assay combined with all types of human and animal cells that can be isolated together with technologies for analysis of cell activation and behavior offers new possibilities for the study of basic mechanisms of normal human development, tissue homeostasis, and disease.

The fluidic culture device can be used to perform screens of the biological activities of any soluble molecular compound. For the screens, cells will be kept in one or several culture chambers of the fluidic culture device. The cells will be subjected to gradients of the compound of interest, or of gradients of multiple factors, but can also be subjected to even concentrations of any type of compound. If a multi-chamber version of the fluidic culture device is used, several gradient and non-gradient conditions can be evaluated on the same chip at the same time and the results thus directly compared.

The fluidic culture device can be used to evaluate molecules that act as inhibitors or activators, i.e. antagonists or agonists, of cell function, including established drugs or new potential drugs. In this context, any type of chemical compound library can be screened for biological activity according to effects elicited in cells grown or kept in the fluidic culture device, and thus evaluated using the culture device. The fluidic culture device can thus be used to screen drugs and to have direct readout of cell responses using live microscopy, or indirect readouts of cell responses by analysis of cells via isolation of the cells after a completed experiment and via further biochemical or cellular analysis.

The platform can be used to check the status of cell lines, to perform quality control of the cell lines that are kept for example in biobanks and cellbanks, to check that they are unaltered with respect to any type of cellular behavior in response to gradients or even levels of any compound, or other specific growth conditions such as level of oxygen, levels of different growth factors, drugs and nutritional factors. For example, human or mouse tumor cells or stem cells used for drug testing and compound screening can subject to quality control by subjecting them at regular time interval to growth conditions, and by comparing the responses. This makes it possible to monitor cell lines kept frozen in cell banks to see that they are keeping their identity and function over time.

The device can be used for industrial production of proteins by cells, bacteria or other protein producing cells, organs, or small organisms. Cells can be maintained in the fluidic culture device and stimulated or not to induce protein production, and produced proteins can thereafter either be collected in the outflow form the system or by harvesting the cells directly from the device followed by protein purification from the cells.

The fluidic culture device can be used to find concentration intervals or minimal concentrations for any given compound that elicits any type of cell response. For example, cells grown at confluency in the fluidic culture device will be exposed to a gradient of a compound, and thereafter analyzed for gene expression or analyzed for activation of different cell signaling pathways. The responses can then be correlated to a specific concentration in order to identify a minimal factor level, i.e. threshold, required to induce a response, and to define the biological responses within a concentration range. The fluidic culture device makes it possible to do this in a 3D tissue, for example in a blood vessel sprout or a cluster of stem cells or cluster of tumor cells.

The fluidic culture device can be fitted into another analytical instrument, such as a microscope, a cell incubator, or any other instruments used to monitor cell responses by various analytical methods to monitor cell behavior in real-time. For example, the IN Cell Analyzer from GE Healthcare can be fitted with the fluidic culture device for functional cell studies. The fluidic culture device is fully compatible with analysis of cells engineered to express fluorescent proteins, or other signals/reportes that can be detected by live imaging or live recording of cells.

The fluidic culture device of the invention can be used not only for stabilizing gradients of at least one substance dissolved or suspended in a liquid or solution entering the fluid channels. In clear contrast, the culture device can also be used in connection with gaseous fluids and the establishment of gas gradients over the culture chamber in the fluidic culture device.

### EXPERIMENTS

The present application study describes a fluidic culture device for graded stimulations of 3-dimensional (3D) organ cultures and complex cell systems. It has been shown that the fluidic culture device can be used to control complex biological processes, exemplified here by angiogenic sprouting and vessel remodeling in embryonic mouse kidneys and embryoid bodies. The new fluidic culture device and related assays for directional sprouting presented here will facilitate research aimed at understanding cell signaling events that control angiogenesis and guidance of vascular tip cells.

### Design and production of casting master

The fluidic culture device was generated by soft lithography using PDMS. The design of the master plate for PDMS casting is outlined in Fig.1. The circular device (diameter 33 mm) encodes a centrally positioned culture chamber for tissue culture (4x3 mm, 850 µm deep), flanked by two flow channels (500 µm wide and 1000 µm deep) used for generation of gradients in the culture chamber. The flow channels are joined in a single outlet. The outer parts of the device holds a network of vacuum channels (200 µm wide and 75 µm deep) that is used to attach the device onto a planar surface. An external vacuum source can be connected to the vacuum grid for firm attachment, but gently pressing the device onto a Petri dish is normally enough to prevent fluid leakage from the system.

The master was fabricated in SU-8 resist (MicroChem, Newton, MA, USA) in three layers with different heights. Structures in the first and third layers were fabricated using standard SU-8 processing, whereas the second and thickest layer (about 850 µm) was processed similar to the method described by Lin et al. [4]. Here, the photoresist was applied onto the wafer at 80 °C to facilitate even distribution of the thick layer, and a scraper was used to spread the SU-8 over the substrate. Care should be taken in this step so that the resist makes contact with the edge of the wafer to prevent the resist from being pulled back during the baking step. The wafer was baked at 120 °C for 8 hours followed by lithography processing. Finally and after completion of the third layer the master was hard-baked at 150 °C for 30 min.

### PDMS casting

PDMS (Sylgard 184 Silicone elastomer kit, Dow Coming) was added to the master and left to polymerize at 70 °C for at least 4 hours. The PDMS substrate was subsequently removed from the master and sterilized in 70 % ethanol. Holes for the inlet and outlet ports and a single hole connecting to the vacuum channels were made using sharp custom-made punchers.

### Tissue seeding

Invasive angiogenic sprouting in three dimensions was studied by embedding organs or clusters of cells in a collagen I matrix composed of Ham's F12 medium (Promocell), 6.26 mM NaOH, 20 mM HEPES, 0.117 % NaHCO₃, 1 % Glutamax-I (Gibco) and 1.5 mg/ml-collagen I (PureCol). The flow channels were sealed prior to casting of the gel by the insertion of PDMS plugs. These were cut out a little wider than the channels (∼7x5x0.6 mm) to prevent the gel from leaking into the flow channels. The gel volume (20 µl) deposited in the culture chamber was approximately twice the size of the chamber volume to account for shrinkage and to make sure that the when the culture system was assembled the gel would be slightly compressed in order to occupy the entire culture chamber. This was possible due the large flexibility of the gel and the compression did not appear to affect the cultures. The collagen gel was left to polymerize in a regular cell incubator at 37 °C and 5 % CO₂ for 2 hours. Organs or cell clusters were deposited into the center of the culture chamber using a Pasteur pipette in the beginning of the polymerization process. The tissue can be carefully positioned with a needle or by tapping the side of the culture chamber. Both organs and cells can be grown in the fluidic culture device in its open configuration with the culture chamber facing upwards for several days by adding cell media to cover the top of the open culture chamber.

### Fluidic system assembly

The fluidic system was finally assembled and made operational. A paper tissue was used to get rid of excess medium and the PDMS plugs were removed with regular forceps. A cover glass or a Petri dish was placed on top of the PDMS structure and light pressure was applied to seal the device. It is important at this stage to make sure that the PDMS surface, except for the culture chamber itself, is completely dry to allow for proper attachment and to prevent bubble formation in the channels. Polyethylene tubing with an inner diameter of 0.8 mm (Intramedic BD, Sparks, MD, USA) was connected to the inlet ports. The outlet port was left unconnected to reduce the resistance in the flow system. When fully assembled, the culture system is referred to as being in its closed configuration. The culture system was inverted prior to further experimentation and real-time analysis through an inverted microscope.

### Growth factors, gradient indicators and gradient generation

Stock solutions of recombinant VEGFA165 (PeproTech), FGF2 (PeproTech) and VEGFC (R&D systems) were stored at -20 °C until further use. A kit for conjugation of proteins to fluorescent dyes was used to couple VEGFA to Alexa Fluor 488 dye (Molecular Probes) to create a fluorescent conjugate (F-VEGFA), according to the manufacturer's instructions. F-VEGFA as well as FITC-dextran of molecular mass 10 kDa (Sigma Aldrich) was used as an indicator of gradients formed in the culture chamber.

Growth factor gradients used for induction of angiogenic sprouting were either a mix of VEGFA, FGF2, and VEGFC (source concentration 100 ng/ml respectively), or gradients of only VEGFA (source concentration 20 ng/ml). Gradients were normally maintained for 48 or 72 hours. Cell culture medium or buffer was pumped through the system at 0.5 µl/min using a syringe pump (Pump 11 PicoPlus, Harvard Apparatus) fitted with gas tight Hamilton syringes (Sigma) connected to the tubing of the inlet ports.

### Gradient generation and characterization

Flow in the culture system was generated by connecting two syringes (connected to a syringe pump) to the channel inlets. One flow channel served as a source for growth factors and gradient indicators, whereas the other flow channel was used as a sink. Gradients were formed in the culture chamber by diffusion of factors between the source and the sink channels. No flow was recorded through the culture chamber when the flow rates in the source and sink channels were kept identical.

The shape of gradients formed in the system will most likely be affected by the diffusive properties of the ligand as well as by the matrix composition and density. Accordingly, differences between FITC-dextran and F-VEGFA gradient shapes were recorded and it is therefore recommended that factors of interest are coupled to fluorescent dyes in order to characterize gradient formation prior to further experimentation.

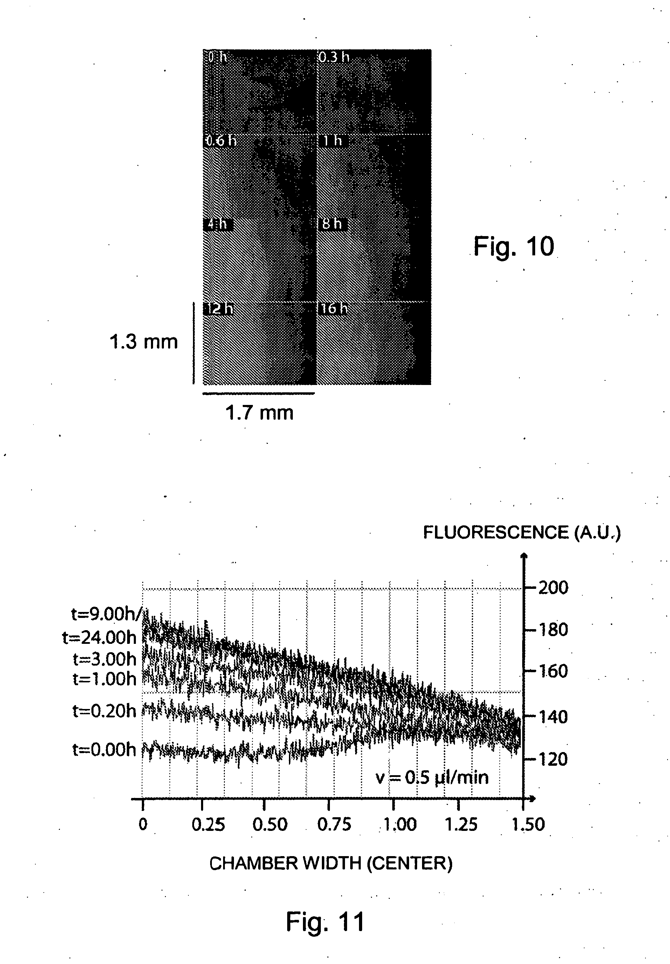

The gradient profile was initially characterized by detection of F-VEGFA. Formation of a linear gradient was achieved approximately 3-4 hours after the onset of flow and after 9 hours the gradient was stable, Figs. 10-12. Fig. 10 illustrates time-lapse series of the central part of the culture chamber (1.7 x 1.3 mm) as a gradient of F-VEGFA is established; gradient formation in the culture chamber was detected by measuring the fluorescent signal. Fig. 11 illustrates time-lapse series of F-VEGFA profiles as the gradient is established. The profiles are recorded at the center of the chamber, indicated by black bar in Fig. 12. Fig. 12 is an illustration of the stable F-VEGFA profile across the full width of the chamber. The affinity of F-VEGFA for the collagen I matrix results in accumulation of growth factor at the edge of the gel. The concentrations specified in the figure represent the amounts of F-VEGFA provided by the source and sink channels.

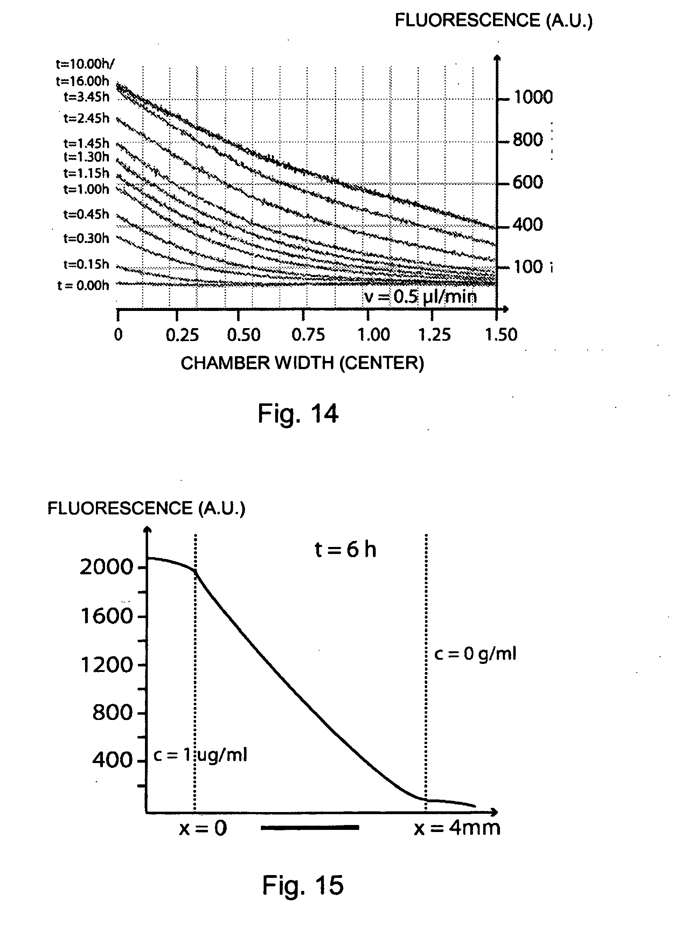

In this context it can be of importance to note that endothelial cells are more sensitive to the gradient shape than to the absolute concentrations. An accumulation of F-VEGFA was seen at the edge of the collagen matrix, suggesting that F-VEGFA interacts weakly with collagen I, see Fig.12. FITC-dextran (molecular mass 10 kDa) on the other hand was shown to have little or no affinity for collagen I as no accumulation was detected, see Fig. 15. A linear gradient of dextran was formed within 2 hours and after approximately 6 hours the gradient was stable, Figs. 13-15. Fig.13 illustrates time-lapse series of the central part of the culture chamber as a gradient of FITC-dextran is established. Fig. 14 shows FITC-dextran profiles recorded at the center of the culture chamber indicated by black bar in Fig. 15, which is an illustration of the stable FITC-dextran profile across the culture chamber.

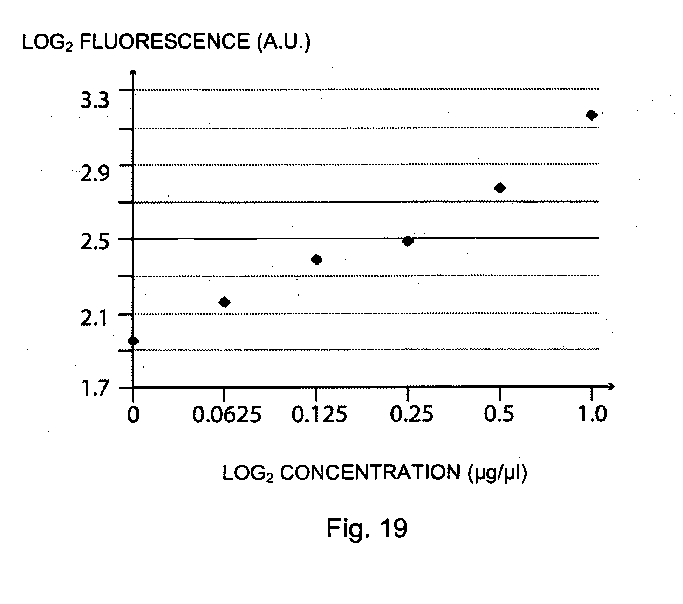

Compared with theoretical calculation based on free diffusion this equilibrium is reached relatively fast. This could at least in part be attributed to the curved shape of the gel/channel interface caused by surface tension which results in a small flow into the gel. The presence of biological material in the chamber did not notably disturb the formation of a FITC-dextran gradient, see Fig. 19 illustrating the relationship between detected fluorescence and FITC-dextran concentration over the interval relevant for this study.

Gradients were in the present study normally maintained for two days. The system is relatively insensitive to perturbations of flow, and syringes may if needed be refilled during the course of an experiment without notably affecting the gradient shape, hence allowing for long experiments.

### Isolation of embryonic mouse kidneys and formation of embryoid bodies

Embryonic kidneys were dissected from E13.5 embryos derived from NMRI mice, and deposited into 20 µl of liquid collagen I matrix in the culture chamber. DMEM-GlutaMax (Invitrogen) supplemented with 0.5 or 5 % serum and 1 % penicillin/streptavidin was used as cell culture medium both for static culture as well as for the fluidic experiments.

Murine embryonic stem cells (line R1) were cultured on growth-arrested mouse embryonic fibroblasts in DMEM-Glutamax (Invitrogen) supplemented with Leukaemia inhibitory factor (LIF, 1000 U/m), 15 % Fetal Bovine Serum (Gibco), 1.2 mM sodium pyruvate, 25 mM HEPES pH 7.4 and 19 mM monothiolglycerol. At day 0, stem cells were trypsinized and resuspended in cell media without LIF and thereafter allowed to differentiate in drops hanging from the lid of a non-adherent culture dish (1200 cells/drop) as previously described [5]. After 4 days, when embryonic stem cells had aggregated to form embryoid bodies (EBs), the drops were collected and the EBs seeded in the culture chamber in between two layers of collagen I gel. Medium containing VEGFA was added to the cultures to give a final concentration of -100 ng/ml. The medium was removed after 24 hours and replaced with growth medium without VEGFA that was than replaced every day. The fluidic device was assembled after 4-6 days of EB culture with the chamber in the open configuration.

### Induction of directional angiogenesis in embryonic kidneys

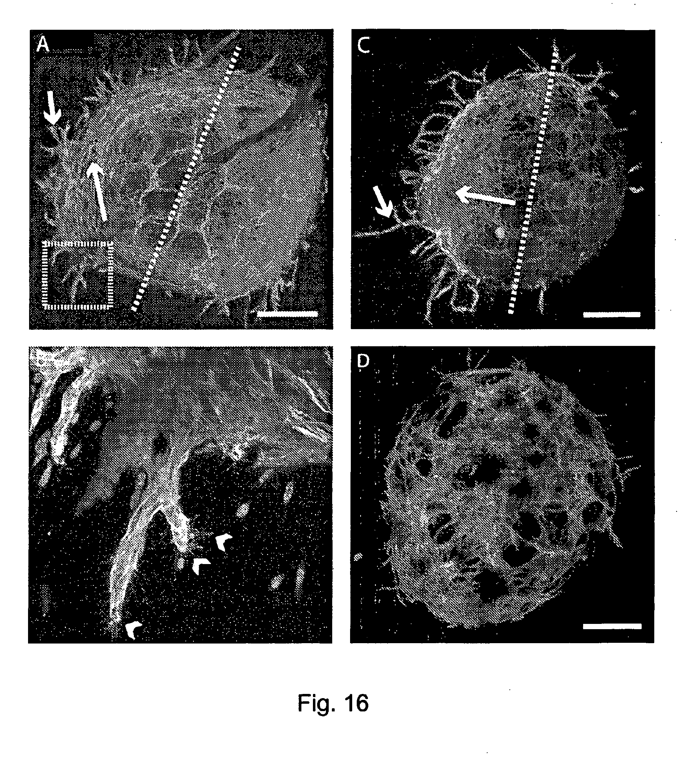

Kidney development has previously been extensively studied by the use of explant cultures of micro-dissected embryonic mouse kidneys. Culture of kidneys isolated at embryonic day (E) 13.5 represents a good model system for the study of angiogenesis and maturation of vascular networks, as the kidney at this point in time has an immature vascular plexus. E13.5 kidneys were deposited into a collagen I matrix at the center of the culture chamber and stimulated with a gradient of mixed angiogenic growth factors, Figs.16A,16B, or with a gradient of VEGFA alone, Fig.16C. After completed experiments the kidneys were retrieved from the device and the vasculature was visualized with antibodies directed against CD31, a known marker for endothelial cells that plays a role in the organization of endothelial junctions.

A combined gradient of VEGFA, FGF2, and VEGFC was shown to be a potent inducer of asymmetrical expansion of the vascular plexus, such that angiogenesis was predominantly induced on the side of the kidney facing high levels of growth factor. Invasive sprouting into the matrix was also more frequent on the side facing high levels of growth factor. The cocktail of growth factors was used on the premises that FGF2 is a survival factor for endothelial cells and that immature blood vessels expresses VEGF-receptor 3 which is a high affinity receptor for VEGFC. However, a gradient of VEGFA alone resulted in similar polarization of the vascular plexus as well as polarized sprouting, Fig. 16C. Non-stimulated kidney explants grown for 48 hours were used for comparison and showed no asymmetrical expansion of the vascular plexus, Fig.16D. Four out of five kidneys asymmetrically stimulated as described above responded with directional sprouting while the fifth kidney did not sprout at all.

The growth of the kidney tissue (i.e. the increase in total kidney tissue volume) during the course of an experiment was not dramatic, and gradients formed were observed to be stable throughout the experiments.

In Figs.16A and 16C, the dotted line indicates the direction in which the concentration of growth factor is approximately constant with higher concentrations to the left in the images. Fig. 16A illustrates embryonic kidney (isolated at E13.5) stimulated with a growth factor gradient containing VEGFA, FGF2, and VEGFC for 48 hours (gradient range 0-100 ng/mL). Effects of the graded stimulation can be seen inside the kidney cortex as an expansion and polarization of the pre-existing vascular plexus (long arrow), as well as by increased invasive sprouting toward higher concentration of factor (short arrow). Fig. 16B is a close-up of sprouts (indicated by box in Fig. 16A) showing tip cells with characteristic protrusions (arrowheads). Fig. 16C illustrates E13.5 kidney stimulated for 48 hours with a VEGFA gradient (gradient range 0-20 ng/mL). Fig. 16D shows control kidney grown for 48 hours in the absence of growth factors. The scale bars represent 200 µm.

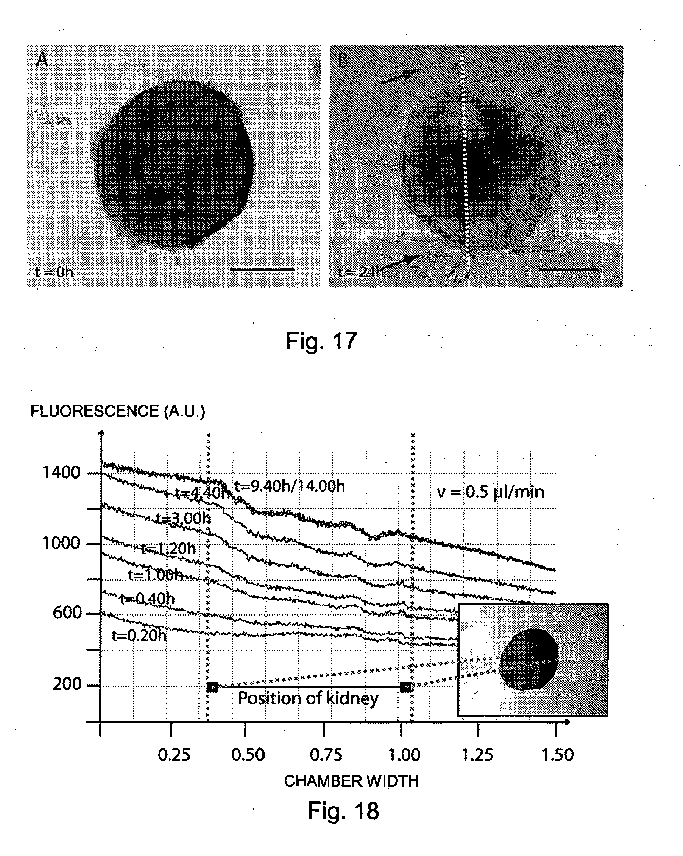

Fig. 18 is a gradient versus time graph showing the formation of a FITC-dextran gradient with a kidney positioned in the chamber. The linearity of the gradient is not greatly disturbed although the slope does not appear as steep as in Fig. 14 due to high autofluorescence of the growth medium. The disturbance at the position of the kidney is due to variation in light absorbance in different parts of the organ.

### Guidance of vascular sprouts in stem cell cultures

Spheroids of differentiating mouse embryonic stem cells, denoted embryoid bodies (EBs), are increasingly being used as a model system for vascular development and angiogenic sprouting. EBs placed in 3D collagen gels have been shown to form highly organized vascular sprouts in response to VEGFA. The EB model is very versatile and powerful as embryonic stem cells can be obtained from all types of genetically engineered mice, and EBs are therefore often used to evaluate gene function in early vasculogenesis and angiogenesis. Sprout formation is however relatively slow in this model; the EBs require a cultivation time in the matrix of at least 4 days before significant sprouting can be detected. For practical reasons, it is therefore advantageous if EBs can be pre-cultivated for at least 4 days before onset of gradient generation, and culturing EBs with the fluidic culture device in the open configuration makes this possible. It is also convenient to set up many fluidic culture devices in parallel, to efficiently perform consecutive experiments without occupying equipment such as pumps and live imaging microscopes during the initial EB growth phase. Further, a fluidic culture device which holds 4 organ culture chambers that will facilitate analysis of samples in parallel (not shown) has also been produced.

EBs grown for an initial 4 days with the device in the open configuration and then stimulated with a gradient of VEGFA for 48 hours showed directional sprouting towards increasing concentrations of VEGFA with a quantitative increase in vessel formation on the side of the EB facing the high end of the growth factor gradient, see Fig. 17.

Mouse embryonic stem cells were aggregated into embryoid bodies (EBs), and grown for 4 days as hanging drops and 4 days in the device prior to onset of the VEGFA gradient. Fig. 17A illustrates EB at day 8 prior to gradient stimulation. Fig. 17B shows the same EB as shown in Fig. 17A at day 9 after 24 hours of asymmetric stimulation with VEGFA. The dotted line indicates the direction in which the concentration of growth factor is approximately constant, with higher VEGFA concentration to the left in the image. Angiogenic sprouts protrude towards higher concentrations of VEGFA (arrows). The scale bars represent 200 µm.

### Immunohistochemistry and microscopy

Collagen gels holding organs or cells were after completed experiments carefully removed from the culture chamber as a single piece, washed briefly in PBS and subsequently fixed with 4 % paraformaldehyde at room temperature for 30 min. The samples where blocked and permeabilized using TNB block buffer (PerkinElmer). Antibodies used were: rat anti-CD31 (BD Pharmingen) and anti-rat Alexa-555 (Invitrogen). Hoechst 33342 (Sigma) was used to visualize cell nuclei. Stained samples were mounted on glass slides in Fluoromount-G (SouthemBiotech) in order to reduce fluorochrome quenching. Images were acquired either using a LSM 510 META confocal microscope (Zeiss) or a Cell Observer System (Zeiss). Pictures of fluorescent molecular gradients were collected every 20 minutes during the course of an experiment using the Cell Observer and Zeiss Axiovision imaging software. The relationship between measured fluorescence intensity and concentration of fluorescent molecule is shown in Fig. 19.

### REFERENCES

[1] International application with publication number WO 2004/034016
[2] Vickerman et al., Lab on a chip, 2008, 8, 1468-1477
[3] Frisk et al., Electrophoresis, 2007, 28, 4705-4712
[4] Lin et al., J. Micromech. Microeng., 2002, 12, 590-597
[5] Jakobsson et al., J Cell Biol, 2007, 177, 751-755

## Claims

1. A fluidic culture device (100) comprising a substrate (101) made of a transparent polymeric material and having a bottom surface (102), a top surface (104) and at least one end surface (106) and comprising:
an open culture chamber (110) present in the form of a hollow in said bottom surface (102) and arranged for housing a culture matrix (200);
a first fluid channel (120) flanking a first side (114) of said open culture chamber (110) and having an inlet (140) provided in said top surface (104) or in an end surface (106) of said substrate (101) and an outlet (160) provided in said top surface (104) or in an end surface (106) of said substrate (101);
a second fluid channel (130) flanking a second, opposite side (115) of said open culture chamber (110) and having an inlet (150) provided in said top surface (104) or in an end surface (106) of said substrate (101) and an outlet (160) provided in said top surface (104) or in an end surface (106) of said substrate (101);
a first removable channel plug (180) removably arranged in a portion of said first fluid channel (120) adjacent to said first side (114) of said open culture chamber (110); and
a second removable channel plug (190) removably arranged in a portion of said second fluid channel (130) adjacent to said second, opposite side (115) of said open culture chamber (110).

2. The device according to claim 1, further comprising a network of open vacuum channels (170) circumferentially provided in said bottom surface (102) relative said open culture chamber (110).

3. The device according to claim 1 or 2, wherein said first fluid channel (120) is an open fluid channel provided in said bottom surface (102) and said second fluid channel (130) is an open fluid channel provided in said bottom surface (102).

4. The device according to any of the claims 1 to 3, wherein said first fluid channel (120) has said inlet (140) provided in said top surface (104), said second fluid channel (130) has said inlet (150) provided in said top surface (104) and said first fluid channel (120) and said second fluid channel (130) have a common outlet (160) provided in said top surface (104).

5. The device according to any of the claims 1 to 4, wherein said bottom surface (102) is not irreversibly bondable to a flat glass or plastic cover surface.

6. The device according to any of the claims 1 to 5, further comprising a culture matrix (200) provided in said open culture chamber (110), whereby said culture matrix (200) being confined by opposite side walls of said open culture chamber (110) and said first removable channel plug (180) and said second removable channel plug (190).

7. A culturing method comprising:
providing a fluidic culture device (100) according to any of the claims 1 to 5;
pouring a liquid gel suspension into said open culture chamber (110) and allowing said gel to polymerize to form a culture matrix (200);
adding a biological sample (210) to said gel prior, during or after polymerization of said gel;
optionally culturing said biological sample (210) in said culture matrix (200) in an open configuration of said fluidic culture device (100) by adding culture medium to said open culture chamber (110) with said culture matrix (200);
removing said first removable channel plug (180) from said first fluid channel (120) and said second removable channel plug (190) from said second fluid channel (130);
reversibly attaching said bottom surface (102) of said substrate (101) to a transparent cover disk (220) to enclose said open culture chamber (110) and said culture matrix (200) and prevent leakage of fluid out of said first fluid channel (120) and said second fluid channel (130); and
connecting a first fluid reservoir (240) containing a fluid to said inlet (140) of said first fluid channel (120) and a second fluid reservoir (250) containing a fluid to said inlet (150) of said second fluid channel (130).

8. The method according to claim 7, wherein said reversibly attaching step comprises reversibly attaching said bottom surface (102) to said transparent cover disk (220) based on a suction pressure caused by a network of vacuum channels (170) circumferentially provided in said bottom surface (102) relative said open culture chamber (110).

9. The method according to claims 7 or 8, further comprising:
removing said transparent cover disk (220) from said substrate (101); and
retrieving said biological sample (210) in said culture matrix (200) from said open culture chamber (110).

10. A culture system (230) comprising:
a fluidic culture device (100) according to any of claims 1-6, wherein first and second removable channel plugs (180, 190) have been removed, comprising a substrate (101) made of a transparent polymeric material and having a bottom surface (102), a top surface (104) and at least one end surface (106) and comprising:
a culture chamber (110) present in the form of a hollow in said bottom surface (102) and housing a culture matrix (200) containing a biological sample (210);
a first fluid channel (120) flanking a first side (114) of said culture chamber (110) and having an inlet (140) provided in said top surface (104) or in an end surface (106) of said substrate (101) and an outlet (160) provided in said top surface (104) or in an end surface (106) of said substrate (101); and
a second fluid channel (130) flanking a second, opposite side (115) of said culture chamber (110) and having an inlet (150) provided in said top surface (104) or in an end surface (106) of said substrate (101) and an outlet (160) provided in said top surface (104) or in an end surface (106) of said substrate (101); and
a transparent cover disk (220) having a flat surface reversibly connected and reversibly attached to said bottom surface (102) to prevent leakage of fluid present in said first fluid channel (120) and said second fluid channel (130).

11. The system according to claim 10, wherein said fluidic culture device (100) further comprises a network of vacuum channels (170) circumferentially provided in said bottom surface (102) relative said culture chamber (110), said transparent cover disk (220) is reversibly attached to said bottom surface (102) by said network of vacuum channels (170).

12. The system according to claim 10 or 11, further comprising:
a first fluid reservoir (240) connected to said inlet (140) of said first fluid channel (120) and containing a fluid having a first concentration of an agent; and
a second fluid reservoir (250) connected to said inlet (150) of said second fluid channel (130) and containing a fluid having a second concentration of said agent, said second concentration being lower than said first concentration.

13. The system according to claim 12, further comprising a pump system for providing a flow of said fluid from said first fluid reservoir (240) into said inlet (140) of said first fluid channel (120) and a flow of said fluid from said second fluid reservoir (250) into said inlet (150) of said second fluid channel (130), wherein a flow rate of said flow of said fluid from said first fluid reservoir (240) into said inlet (140) of said first fluid channel (120) being substantially equal to a flow rate of said flow of said fluid from said second fluid reservoir (250) into said inlet (150) of said second fluid channel (130) so that no net flow of said fluid is present through said culture chamber (110).

14. The system according to any of the claims 10 to 13, wherein said inlets (140, 150) of said first fluid channel (120) and said second fluid channel (130) are provided in said top surface (104) and said culture system (230) further comprising a cover (260) attached to said top surface (104) and comprising:
a transparent window (265) aligned with said culture chamber (110);
a first connecting structure (267) having a channel connecting part (264) extending from a bottom surface of said cover (260) into said inlet (140) of said first fluid channel (120), a tube connecting part (262) aligned with said channel connecting part (264) and extending from a top surface of said cover (260), and a bore (266) through said channel connecting part (264) and said tube connecting part (262); and
a second connecting structure (267) having a channel connecting part (264) extending from said bottom surface of said cover (101) into said inlet (150) of said second fluid channel (130), a tube connecting part (262) aligned with said channel connecting part (264) and extending from said top surface of said cover (260), and a bore (266) through said channel connecting part (264) and said tube connecting part (262).

15. A method of producing a fluidic culture device (100) comprising:
providing a casting master (1) having a chamber defining structure (10) flanked on either sides by respective channel defining structures (20, 30);
adding a transparent polymeric material to said casting master (1) and allowing said polymeric material to polymerize to form a transparent substrate (101) with an open culture chamber (110) present in the form of a hollow in a bottom surface (102) of said substrate (101) and arranged for housing a culture matrix (200), a first fluid channel (120) flanking a first side (114) of said open culture chamber (110) and having an inlet (140) provided in a top surface (104) of said substrate (101) or in an end surface (106) of said substrate (101) and an outlet (160) provided in said top surface (104) or in an end surface (106) of said substrate (101), and a second fluid channel (130) flanking a second, opposite side (115) of said open culture chamber (110) and having an inlet (150) provided in said top surface (104) or in an end surface (106) of said substrate (101) and an outlet (160) provided in said top surface (104) or in an end surface (106) of said substrate (101);
removing said substrate (101) from said casting master (1);
removably arranging a first removable channel plug (180) in a portion of said first fluid channel (120) adjacent to said first side (114) of said open culture chamber (110) and a second removable channel plug (190) in a portion of said second fluid channel (130) adjacent to said second, opposite side (115) of said open culture chamber (110).

## Patentansprüche

1. Fluidkulturvorrichtung (100), die ein Substrat (101) umfasst, das aus einem durchsichtigen Polymermaterial hergestellt ist und eine untere Fläche (102), eine obere Fläche (104) und mindestens eine Endfläche (106) aufweist, und Folgendes umfasst:
eine offene Kulturkammer (110), die in Form einer Aushöhlung in der unteren Fläche (102) vorhanden und angeordnet ist, eine Kulturmatrix (200) aufzunehmen;
einen ersten Fluidkanal (120), der an einer ersten Seite (114) der offenen Kulturkammer (110) liegt und einen Einlass (140), der in der oberen Fläche (104) oder in einer Endfläche (106) des Substrats (101) vorgesehen ist, und einen Auslass (160) aufweist, der in der oberen Fläche (104) oder in einer Endfläche (106) des Substrats (101) vorgesehen ist;
einen zweiten Fluidkanal (130), der an einer zweiten gegenüberliegenden Seite (115) der offenen Kulturkammer (110) liegt und einen Einlass (150), der in der oberen Fläche (104) oder in einer Endfläche (106) des Substrats (101) vorgesehen ist, und einen Auslass (160) aufweist, der in der oberen Fläche (104) oder in einer Endfläche (106) des Substrats (101) vorgesehen ist;
einen ersten herausnehmbaren Kanalstopfen (180), der herausnehmbar in einem Abschnitt des ersten Fluidkanals (120) angrenzend an der ersten Seite (114) der offenen Kulturkammer (110) angeordnet ist; und
einen zweiten herausnehmbaren Kanalstopfen (190), der herausnehmbar in einem Abschnitt des zweiten Fluidkanals (130) angrenzend an der zweiten gegenüberliegenden Seite (115) der offenen Kulturkammer (110) angeordnet ist.

2. Vorrichtung nach Anspruch 1, ferner umfassend ein Netz aus offenen Vakuumkanälen (170), die in der unteren Fläche (102) bezogen auf die offene Kulturkammer (110) in Umfangsrichtung vorgesehen sind.

3. Vorrichtung nach Anspruch 1 oder 2, wobei der erste Fluidkanal (120) ein offener Fluidkanal ist, der in der unteren Fläche (102) vorgesehen ist, und der zweite Fluidkanal (130) ein offener Fluidkanal ist, der in der unteren Fläche (102) vorgesehen ist.

4. Vorrichtung nach einem der Ansprüche 1 bis 3, wobei der Einlass (140) des ersten Fluidkanals (120) in der oberen Fläche (104) vorgesehen ist, der Einlass (150) des zweiten Fluidkanals (130) in der oberen Fläche (104) vorgesehen ist und der erste Fluidkanal (120) und der zweite Fluidkanal (130) einen gemeinsamen Auslass (160) aufweisen, der in der oberen Fläche (104) vorgesehen ist.

5. Vorrichtung nach einem der Ansprüche 1 bis 4, wobei die untere Fläche (102) nicht unumkehrbar mit einer ebenen Glas- oder Kunststoffdeckfläche verbindbar ist.

6. Vorrichtung nach einem der Ansprüche 1 bis 5, ferner umfassend eine Kulturmatrix (200), die in der offenen Kulturkammer (110) vorgesehen ist, wodurch die Kulturmatrix (200) durch gegenüberliegende Seitenwände der offenen Kulturkammer (110) und den ersten herausnehmbaren Kanalstopfen (180) und den zweiten herausnehmbaren Kanalstopfen (190) begrenzt ist.

7. Kultivierungsverfahren, umfassend:
Bereitstellen einer Fluidkulturvorrichtung (100) nach einem der Ansprüche 1 bis 5;
Gießen einer flüssigen Gelsuspension in die offene Kulturkammer (110) und Polymerisierenlassen des Gels, um eine Kulturmatrix (200) zu bilden;
Zugeben einer biologischen Probe (210) zu dem Gel vor, während oder nach der Polymerisierung des Gels;
optional Kultivieren der biologischen Probe (210) in der Kulturmatrix (200) in einer offenen Anordnung der Fluidkulturvorrichtung (100) durch Zugeben von Nährmedium in die offene Kulturkammer (110) mit der Kulturmatrix (200);
Herausnehmen des ersten herausnehmbaren Kanalstopfens (180) aus dem ersten Fluidkanal (120) und des zweiten herausnehmbaren Kanalstopfens (190) aus dem zweiten Fluidkanal (130);
umkehrbares Befestigen der unteren Fläche (102) des Substrats (101) an einer durchsichtigen Deckscheibe (220), um die offene Kulturkammer (110) und die Kulturmatrix (200) zu umschließen und das Herauslaufen von Fluid aus dem ersten Fluidkanal (120) und dem zweiten Fluidkanal (130) zu verhindern; und
Verbinden eines ersten Fluidvorratsbehälters (240), der ein Fluid enthält, mit dem Einlass (140) des ersten Fluidkanals (120) und eines zweiten Fluidvorratsbehälters (250), der ein Fluid enthält, mit dem Einlass (150) des zweiten Fluidkanals (130).

8. Verfahren nach Anspruch 7, wobei der Schritt des umkehrbaren Befestigens das umkehrbare Befestigen der unteren Fläche (102) an der durchsichtigen Deckscheibe (220) basierend auf einem Saugdruck umfasst, der von einem Netz aus Vakuumkanälen (170) bewirkt wird, die in der unteren Fläche (102) bezogen auf die offene Kulturkammer (110) in Umfangsrichtung vorgesehen sind.

9. Verfahren nach Anspruch 7 oder 8, ferner umfassend:
Abnehmen der durchsichtigen Deckscheibe (220) von dem Substrat (101); und
Entnehmen der biologischen Probe (210) in der Kulturmatrix (200) aus der offenen Kulturkammer (110).

10. Kultursystem (230), umfassend:
eine Fluidkulturvorrichtung (100) nach einem der Ansprüche 1 bis 6, bei der ein erster und zweiter herausnehmbarer Kanalstopfen (180, 190) herausgenommen wurden, die ein Substrat (101) umfasst, das aus einem durchsichtigen Polymermaterial hergestellt ist und eine untere Fläche (102), eine obere Fläche (104) und mindestens eine Endfläche (106) aufweist, und Folgendes umfasst:
eine Kulturkammer (110), die in Form einer Aushöhlung in der unteren Fläche (102) vorhanden ist und eine Kulturmatrix (200), die eine biologische Probe (210) enthält, aufnimmt;
einen ersten Fluidkanal (120), der an einer ersten Seite (114) der Kulturkammer (110) liegt und einen Einlass (140), der in der oberen Fläche (104) oder in einer Endfläche (106) des Substrats (101) vorgesehen ist, und einen Auslass (160) aufweist, der in der oberen Fläche (104) oder in einer Endfläche (106) des Substrats (101) vorgesehen ist; und
einen zweiten Fluidkanal (130), der an einer zweiten gegenüberliegenden Seite (115) der Kulturkammer (110) liegt und einen Einlass (150), der in der oberen Fläche (104) oder in einer Endfläche (106) des Substrats (101) vorgesehen ist, und einen Auslass (160) aufweist, der in der oberen Fläche (104) oder in einer Endfläche (106) des Substrats (101) vorgesehen ist; und
eine durchsichtige Deckscheibe (220), die eine ebene Fläche aufweist, umkehrbar mit der unteren Fläche (102) verbunden und umkehrbar daran befestigt ist, um das Herauslaufen von Fluid, das sich in dem ersten Fluidkanal (120) und dem zweiten Fluidkanal (130) befindet, zu verhindern.

11. System nach Anspruch 10, wobei die Fluidkulturvorrichtung (100) ferner ein Netz aus Vakuumkanälen (170) umfasst, die in der unteren Fläche (102) bezogen auf die Kulturkammer (110) in Umfangsrichtung vorgesehen sind, die durchsichtige Deckscheibe (220) durch das Netz aus Vakuumkanälen (170) umkehrbar an der unteren Fläche (102) befestigt ist.

12. System nach Anspruch 10 oder 11, ferner umfassend:
einen ersten Fluidvorratsbehälter (240), der mit dem Einlass (140) des ersten Fluidkanals (120) verbunden ist und ein Fluid enthält, das eine erste Konzentration eines Mittels aufweist; und
einen zweiten Fluidvorratsbehälter (250), der mit dem Einlass (150) des zweiten Fluidkanals (130) verbunden ist und ein Fluid enthält, das eine zweite Konzentration des Mittels aufweist, wobei die zweite Konzentration geringer ist als die erste Konzentration.

13. System nach Anspruch 12, ferner umfassend ein Pumpsystem zum Bereitstellen eines Stroms des Fluids aus dem ersten Fluidvorratsbehälter (240) in den Einlass (140) des ersten Fluidkanals (120) und eines Stroms des Fluids aus dem zweiten Fluidvorratsbehälter (250) in den Einlass (150) des zweiten Fluidkanals (130), wobei ein Durchfluss des Stroms des Fluids aus dem ersten Fluidvorratsbehälter (240) in den Einlass (140) des ersten Fluidkanals (120) im Wesentlichen einem Durchfluss des Stroms des Fluids aus dem zweiten Fluidvorratsbehälter (250) in den Einlass (150) des zweiten Fluidkanals (130) entspricht, sodass durch die Kulturkammer (110) kein Nettofluss des Fluids vorhanden ist.

14. System nach einem der Ansprüche 10 bis 13, wobei die Einlässe (140, 150) des ersten Fluidkanals (120) und des zweiten Fluidkanals (130) in der oberen Fläche (104) vorgesehen sind und das Kultursystem (230) ferner eine Abdeckung (260) umfasst, die an der oberen Fläche (104) befestigt ist und Folgendes umfasst:
ein durchsichtiges Fenster (265), das nach der Kulturkammer (110) ausgerichtet ist;
eine erste Verbindungsstruktur (267), die ein Kanalverbindungsteil (264) aufweist, das sich von einer unteren Fläche der Abdeckung (260) in den Einlass (140) des ersten Fluidkanals (120) erstreckt, ein Röhrenverbindungsteil (262), das nach dem Kanalverbindungsteil (264) ausgerichtet ist und sich von einer oberen Fläche der Abdeckung (260) aus erstreckt, und eine Bohrung (266) durch das Kanalverbindungsteil (264) und das Röhrenverbindungsteil (262); und
eine zweite Verbindungsstruktur (267), die ein Kanalverbindungsteil (264) aufweist, das sich von der unteren Fläche der Abdeckung (101) in den Einlass (150) des zweiten Fluidkanals (130) erstreckt, ein Röhrenverbindungsteil (262), das nach dem Kanalverbindungsteil (264) ausgerichtet ist und sich von der oberen Fläche der Abdeckung (260) aus erstreckt, und eine Bohrung (266) durch das Kanalverbindungsteil (264) und das Röhrenverbindungsteil (262).

15. Verfahren zum Herstellen einer Fluidkulturvorrichtung (100), umfassend:
Bereitstellen einer Gießvorlage (1), die eine kammerdefinierende Struktur (10) aufweist, die auf beiden Seiten von entsprechenden kanaldefinierenden Strukturen (20, 30) flankiert wird;
Zugeben eines durchsichtigen Polymermaterials zu der Gießvorlage (1) und Polymerisierenlassen des Polymermaterials, um ein durchsichtiges Substrat (101) zu formen, mit einer offenen Kulturkammer (110), die in Form einer Aushöhlung in einer unteren Fläche (102) des Substrats (101) vorhanden und angeordnet ist, eine Kulturmatrix (200) aufzunehmen, einem ersten Fluidkanal (120), der an einer ersten Seite (114) der offenen Kulturkammer (110) liegt und einen Einlass (140), der in einer oberen Fläche (104) des Substrats (101) oder in einer Endfläche (106) des Substrats (101) vorgesehen ist, und einen Auslass (160) aufweist, der in der oberen Fläche (104) oder in einer Endfläche (106) des Substrats (101) vorgesehen ist, und einem zweiten Fluidkanal (130), der an einer zweiten gegenüberliegenden Seite (115) der offenen Kulturkammer (110) liegt und einen Einlass (150), der in der oberen Fläche (104) oder in einer Endfläche (106) des Substrats (101) vorgesehen ist, und einen Auslass (160) aufweist, der in der oberen Fläche (104) oder in einer Endfläche (106) des Substrats (101) vorgesehen ist;
Entnehmen des Substrats (101) aus der Gießvorlage (1);
herausnehmbares Anordnen eines ersten herausnehmbaren Kanalstopfens (180) in einem Abschnitt des ersten Fluidkanals (120) angrenzend an der ersten Seite (114) der offenen Kulturkammer (110) und eines zweiten herausnehmbaren Kanalstopfens (190) in einem Abschnitt des zweiten Fluidkanals (130) angrenzend an der zweiten gegenüberliegenden Seite (115) der offenen Kulturkammer (110).

## Revendications

1. Dispositif de culture fluidique (100) comprenant un substrat (101) fabriqué en un matériau polymère transparent et présentant une surface inférieure (102), une surface supérieure (104) et au moins une surface d'extrémité (106) et comprenant :
une chambre de culture ouverte (110) présente sous forme d'une cavité dans ladite surface inférieure (102) et agencée pour recevoir une matrice de culture (200) ;
un premier canal de fluide (120) bordant un premier côté (114) de ladite chambre de culture ouverte (110) et présentant une entrée (140) prévue dans ladite surface supérieure (104) ou dans une surface d'extrémité (106) dudit substrat (101) et une sortie (160) prévue dans ladite surface supérieure (104) ou dans une surface d'extrémité (106) dudit substrat (101) ;
un second canal de fluide (130) bordant un second côté opposé (115) de ladite chambre de culture ouverte (110) et présentant une entrée (150) prévue dans ladite surface supérieure (104) ou dans une surface d'extrémité (106) dudit substrat (101) et une sortie (160) prévue dans ladite surface supérieure (104) ou dans une surface d'extrémité (106) dudit substrat (101) ;
un premier bouchon de canal amovible (180) agencé de manière amovible dans une partie dudit premier canal de fluide (120) adjacente audit premier côté (114) de ladite chambre de culture ouverte (110) ; et
un second bouchon de canal amovible (190) agencé de manière amovible dans une partie dudit second canal de fluide (130) adjacente audit second côté opposé (115) de ladite chambre de culture ouverte (110).

2. Dispositif selon la revendication 1, comprenant en outre un réseau de canaux à vide ouverts (170) prévus de manière circonférentielle dans ladite surface inférieure (102) par rapport à ladite chambre de culture ouverte (110).

3. Dispositif selon la revendication 1 ou 2, dans lequel ledit premier canal de fluide (120) est un canal de fluide ouvert prévu dans ladite surface inférieure (102) et ledit second canal de fluide (130) est un canal de fluide ouvert prévu dans ladite surface inférieure (102).

4. Dispositif selon l'une quelconque des revendications 1 à 3, dans lequel ledit premier canal de fluide (120) présente ladite entrée (140) prévue dans ladite surface supérieure (104), ledit second canal de fluide (130) présente ladite entrée (150) prévue dans ladite surface supérieure (104) et ledit premier canal de fluide (120) et ledit second canal de fluide (130) présentent une sortie commune (160) prévue dans ladite surface supérieure (104).

5. Dispositif selon l'une quelconque des revendications 1 à 4, dans lequel ladite surface inférieure (102) ne peut pas adhérer de manière irréversible à une surface de recouvrement plate en verre ou en plastique.

6. Dispositif selon l'une quelconque des revendications 1 à 5, comprenant en outre une matrice de culture (200) prévue dans ladite chambre de culture ouverte (110), ce par quoi ladite matrice de culture (200) est enfermée par des parois latérales opposées de ladite chambre de culture ouverte (110) et ledit premier bouchon de canal amovible (180) et ledit second bouchon de canal amovible (190).

7. Procédé de culture comprenant :
prévoir un dispositif de culture fluidique (100) selon l'une quelconque des revendications 1 à 5 ;
verser une suspension de gel liquide dans ladite chambre de culture ouverte (110) et permettre audit gel de polymériser pour former une matrice de culture (200) ;
ajouter un échantillon biologique (210) audit gel avant, pendant ou après la polymérisation dudit gel ;
en option faire une culture dudit échantillon biologique (210) dans ladite matrice de culture (200) dans une configuration ouverte dudit dispositif de culture fluidique (100) en ajoutant un milieu de culture à ladite chambre de culture ouverte (110) avec ladite matrice de culture (200) ;
retirer ledit premier bouchon de canal amovible (180) dudit premier canal de fluide (120) et ledit second bouchon de canal amovible (190) dudit second canal de fluide (130) ;
fixer de manière réversible ladite surface inférieure (102) dudit substrat (101) à un disque de recouvrement transparent (220) pour enfermer ladite chambre de culture ouverte (110) et ladite matrice de culture (200) et empêcher une fuite de fluide hors dudit premier canal de fluide (120) et dudit second canal de fluide (130) ; et
connecter un premier réservoir de fluide (240) contenant un fluide à ladite entrée (140) dudit premier canal de fluide (120) et un second réservoir de fluide (250) contenant un fluide à ladite entrée (150) dudit second canal de fluide (130).

8. Procédé selon la revendication 7, dans lequel ladite étape de fixation de manière réversible comprend la fixation de manière réversible de ladite surface inférieure (102) audit disque de recouvrement transparent (220) sur la base d'une pression d'aspiration causée par un réseau de canaux à vide (170) prévus de manière circonférentielle dans ladite surface inférieure (102) par rapport à ladite chambre de culture ouverte (110).

9. Procédé selon la revendication 7 ou 8, comprenant en outre :
retirer ledit disque de recouvrement transparent (220) dudit substrat (101) ; et
extraire ledit échantillon biologique (210) dans ladite matrice de culture (200) de ladite chambre de culture ouverte (110).

10. Système de culture (230), comprenant :
un dispositif de culture fluidique (100) selon l'une quelconque des revendications 1 à 6, dans lequel des premier et second bouchons de canal amovibles (180, 190) ont été retirés, comprenant un substrat (101) fabriqué en un matériau polymère transparent et présentant une surface inférieure (102), une surface supérieure (104) et au moins une surface d'extrémité (106) et comprenant :
une chambre de culture (110) présente sous forme d'une cavité dans ladite surface inférieure (102) et recevant une matrice de culture (200) contenant un échantillon biologique (210) ;
un premier canal de fluide (120) bordant un premier côté (114) de ladite chambre de culture (110) et présentant une entrée (140) prévue dans ladite surface supérieure (104) ou dans une surface d'extrémité (106) dudit substrat (101) et une sortie (160) prévue dans ladite surface supérieure (104) ou dans une surface d'extrémité (106) dudit substrat (101) ; et
un second canal de fluide (130) bordant un second côté opposé (115) de ladite chambre de culture (110) et présentant une entrée (150) prévue dans ladite surface supérieure (104) ou dans une surface d'extrémité (106) dudit substrat (101) et une sortie (160) prévue dans ladite surface supérieure (104) ou dans une surface d'extrémité (106) dudit substrat (101) ; et
un disque de recouvrement transparent (220) présentant une surface plate connectée de manière réversible et fixée de manière réversible à ladite surface inférieure (102) pour empêcher une fuite de fluide présent dans ledit premier canal de fluide (120) et ledit second canal de fluide (130).

11. Système selon la revendication 10, dans lequel ledit dispositif de culture fluidique (100) comprend en outre un réseau de canaux à vide (170) prévus de manière circonférentielle dans ladite surface inférieure (102) par rapport à ladite chambre de culture (110), ledit disque de recouvrement transparent (220) est fixé de manière réversible à ladite surface inférieure (102) par ledit réseau de canaux à vide (170).

12. Système selon la revendication 10 ou 11, comprenant en outre :
un premier réservoir de fluide (240) connecté à ladite entrée (140) dudit premier canal de fluide (120) et contenant un fluide présentant une première concentration d'un agent ; et
un second réservoir de fluide (250) connecté à ladite entrée (150) dudit second canal de fluide (130) et contenant un fluide présentant une seconde concentration dudit agent, ladite seconde concentration étant inférieure à ladite première concentration.

13. Système selon la revendication 12, comprenant en outre un système de pompe pour mettre à disposition un écoulement dudit fluide dudit premier réservoir de fluide (240) dans ladite entrée (140) dudit premier canal de fluide (120) et un écoulement dudit fluide dudit second réservoir de fluide (250) dans ladite entrée (150) dudit second canal de fluide (130), dans lequel un débit dudit écoulement dudit fluide dudit premier réservoir de fluide (240) dans ladite entrée (140) dudit premier canal de fluide (120) est essentiellement égal à un débit dudit écoulement dudit fluide dudit second réservoir de fluide (250) dans ladite entrée (150) dudit second canal de fluide (130) de sorte qu'aucun écoulement net dudit fluide n'est présent à travers ladite chambre de culture (110).

14. Système selon l'une quelconque des revendications 10 à 13, dans lequel lesdites entrées (140, 150) dudit premier canal de fluide (120) et dudit second canal de fluide (130) sont prévues dans ladite surface supérieure (104) et ledit système de culture (230) comprenant en outre un couvercle (260) fixé à ladite surface supérieure (104) et comprenant :
une fenêtre transparente (265) alignée avec ladite chambre de culture (110) ;
une première structure de connexion (267) présentant une pièce de connexion de canal (264) s'étendant d'une surface inférieure dudit couvercle (260) dans ladite entrée (140) dudit premier canal de fluide (120), une pièce de connexion de tube (262) alignée avec ladite pièce de connexion de canal (264) et s'étendant d'une surface supérieure dudit couvercle (260), et un alésage (266) à travers ladite pièce de connexion de canal (264) et ladite pièce de connexion de tube (262) ; et
une seconde structure de connexion (267) présentant une pièce de connexion de canal (264) s'étendant de ladite surface inférieure dudit couvercle (101) dans ladite entrée (150) dudit second canal de fluide (130), une pièce de connexion de tube (262) alignée avec ladite pièce de connexion de canal (264) et s'étendant de ladite surface supérieure dudit couvercle (260), et un alésage (266) à travers ladite pièce de connexion de canal (264) et ladite pièce de connexion de tube (262).

15. Procédé de production d'un dispositif de culture fluidique (100) comprenant :
prévoir un gabarit de moulage (1) présentant une structure de définition de chambre (10) bordée de chaque côté de structures de définition de canal respectives (20, 30) ;
ajouter un matériau polymère transparent audit gabarit de moulage (1) et permettre audit matériau polymère de polymériser pour former un substrat transparent (101) avec une chambre de culture ouverte (110) présente sous forme d'une cavité dans une surface inférieure (102) dudit substrat (101) et agencée pour recevoir une matrice de culture (200), une premier canal de fluide (120) bordant un premier côté (114) de ladite chambre de culture ouverte (110) et présentant une entrée (140) prévue dans une surface supérieure (104) dudit substrat (101) ou dans une surface d'extrémité (106) dudit substrat (101) et une sortie (160) prévue dans ladite surface supérieure (104) ou dans une surface d'extrémité (106) dudit substrat (101), et un second canal de fluide (130) bordant un second côté opposé (115) de ladite chambre de culture ouverte (110) et présentant une entrée (150) prévue dans ladite surface supérieure (104) ou dans une surface d'extrémité (106) dudit substrat (101) et une sortie (160) prévue dans ladite surface supérieure (104) ou dans une surface d'extrémité (106) dudit substrat (101) ;
retirer ledit substrat (101) dudit gabarit de moulage (1) ;
agencer de manière amovible un premier bouchon de canal amovible (180) dans une partie dudit premier canal de fluide (120) adjacente audit premier côté (114) de ladite chambre de culture ouverte (110) et un second bouchon de canal amovible (190) dans une partie dudit second canal de fluide (130) adjacente audit second côté opposé (115) de ladite chambre de culture ouverte (110).
